# EUROPEAN PATENT SPECIFICATION

(11) **EP 1 248 646 B1**
(45) Date of publication and mention of the grant of the patent: **13.02.2008**
(21) Application number: 00991286.6
(22) Date of filing: 20.12.2000
(51) Int. Cl.: A61K 39/002, A61K 48/00

(54) **COMPOSITIONS AND VACCINES CONTAINING ANTIGEN(S) OF CRYPTOSPORIDIUM PARVUM AND OF ANOTHER ENTERIC PATHOGEN**
MINDESTENS EIN CRYPTOSPORIDIUM PARVUM-ANTIGEN UND MINDESTENS EIN ANTIGEN EINES ANDEREN DARMKRANKHEITSERREGERS ENTHALTENDE ZUSAMMENSETZUNGEN UND IMPFSTOFFE
COMPOSITIONS ET VACCINS CONTENANT DES ANTIGENES DE CRYPTOSPORIDIUM PARVUM ET D'UN AUTRE PATHOGENE

(30) Priority: 21.12.1999 US 171399 P
(43) Date of publication of application: 16.10.2002
(73) Proprietor: MERIAL, 69358 Lyon Cédex 07 (FR)
(72) Inventor: AUDONNET, Jean-Christophe, F-69005 Lyon (FR); GALLO, Guillermo, Athens, GA 30601 (US)
(74) Representative: Harding, Charles Thomas
(86) International application number: PCT/EP2000/013387
(87) International publication number: WO 2001/045735

(56) References cited:
- WO-A-98/06430
- WO-A-98/07320
- US-A- 5 591 434
- PERRYMAN, LANCE E. ET AL: "Protection of calves against cryptosporidiosis with immune bovine colostrum induced by a Cryptosporidium parvum recombinant protein" VACCINE (1999), 17(17), 2142-2149 , XP004164999
- SAGODIRA, SERGE ET AL: "Protection of kids against Cryptosporidium parvum infection after immunization of dams with CP15 -DNA" VACCINE (1999), 17(19), 2346-2355 , XP004168965
- DE GRAAF D C ET AL: "Speculation on whether a vaccine against cryptosporidiosis is a reality or fantasy." INTERNATIONAL JOURNAL FOR PARASITOLOGY, (1999 AUG) 29 (8) 1289-306. REF: 124 , XP001000163
- HARP, J. A. ET AL: "Strategies for the control of Cryptosporidium parvum infection in calves" J. DAIRY SCI. (1998), 81(1), 289-294 , XP001000159
- KALINNA, BERND H.: "DNA vaccines for parasitic infections" IMMUNOL. CELL BIOL. (1997), 75(4), 370-375 , XP001005498
- BOLAND, WAYNE (1) ET AL: "Interactions between vaccination, failure of passive transfer, and diarrhea in beef calves." AGRI-PRACTICE, (1995) VOL. 16, NO. 4, PP. 25-28. , XP001005673
- Jenkins M. et al: (1995) Vaccine 13:1658-1664
- Jenkins M.C. et al: (1998) Vaccine 17:2453-2460
- Iochmann S. et al: (1999) Microbial Pathogenesis 26:307-315
- Jenkins M.C. et al: (1999) Clin. Diagn. Lab. Immunol. 6:912-920

## Description

### FIELD OF THE INVENTION

The invention relates to antigen(s)/epitope(s) of *Cryptosporidium parvum* and enteric pathogens (such as other enteric pathogens), compositions and methods comprising or using the same for eliciting an immune response against, or for prevention, treatment, or control of *Cryptosporidium parvum* and/or enteric infections, and uses thereof.

There is also mentioned herein methods and/or compositions, and/or uses of such compositions or components thereof in formulating such compositions, for eliciting an immune response against and/or for the prevention and/or treatment and/or control of enteric infections in animals, for instance mammals, such as bovines, felines, canines or equines or species thereof.

The invention relates also to compositions, and/or uses of such compositions or components thereof in formulating such compositions, for eliciting an immune response against and/or for the prevention and/or treatment and/or control of infection by *Cryptosporidium parvum*.

There is also mentioned herein the concurrent use of a monovalent *Cryptosporidium parvum* vaccine with enteric, e.g. bovine enteric (e.g., rota/coronavirus, *E. coli*) vaccines and/or use of a combination vaccine containing *Cryptosporidium parvum* + rota/coronavirus, *E. coli,* as well as to preventing, controlling or treating or eliciting an immune response to reduce exacerbation of enteric, e.g., bovine enteric, diseases due to co-infection with *Cryptosporidium parvum.* The immunity induced by vaccination against *Cryptosporidium parvum,* can significantly reduce the severity of the disease induced by herein mentioned enteric pathogens. A combination vaccine containing *Cryptosporidium parvum* is useful for a more complete prevention of multietiological enteric disease in newborn animals, such as calves, caused by rota and coronaviruses and *E. coli* K99 and F41.

There is also mentioned herein the effects of *Cryptosporidum parvum* co-infection on other enteric, e.g., bovine enteric, pathogens. *Cryptosporidium parvum* is commonly found in the feces of newborn animals such as mammals, e.g., calves. *Cryptosporidium parvum* is able to produce clinical signs of enteric disease by itself, regardless of the presence or absence of other potentially pathogenic viruses and bacteria in the gut. Viruses, such as coronavirus, and bacteria, such as *E. coli* e.g., F41, that have been recognized in the field as very pathogenic are not able to cause important clinical signs of disease in experimental challenge models. Thus, the invention can relate to addressing the co-infection of cattle with *Cryptosporidium parvum* as that co-infection can exacerbate the disease caused by other enteric pathogens such as coronavirus, rotavirus, and *E. coli* e.g., F41.

Various documents are cited in this text. Citations in the text can be by way of a citation to a document in the reference list, e.g., by way of an anthor(s) and document year citation to a document listed in the reference list, or by full citation in the text to a document that may or may not also be listed in the reference list.

There is no admission that any of the various documents cited in this text are prior art as to the present invention. Any document having as an author or inventor person or persons named as an inventor herein is a document that is not by another as to the inventive entity herein.

### BACKGROUND OF THE INVENTION

Bovine enteric disease is the result of an enteropathogenic intestinal infection that most often manifests itself in some form of diarrhea. This disease, also commonly referred to as neonatal calf diarrhea, is responsible for substantial economic loss in the farming industry. The morbidity of the calves, together with the need for therapeutic intervention and the possible long term detrimental effects on the animals, are the main factors responsible for the economic burden on the farmer. One estimate indicates that neonatal calf diarrhea is responsible for about 75% of the death of dairy calves under 3-weeks of age. Radostits, OM, et al., Herd Health Food Animal Production Medicine, 2nd ed., Sounders, Philadelphia, pp. 184-213, 1994. The management of neonatal calf diarrhea is difficult for multiple reasons, some of the most important which include: (1) the involvement of multiple agents in the pathogenesis of the disease; (2) the nonspecificity of clinical signs; (3) the finding that some infections can be asymptomatic; and, (4) the involvement of host factors such as nutrition and endogenous immunity. Moon, HW, et al., JAVMA 173 (5): 577 - 583 (1978). Viring, S. et al., Acta Vet. Scand. 34: 271- 279 (1999).

Developing a strategy to prevent or treat bovine enteric disease has been very difficult since while it is known that multiple enteropathogens are present during the infection, it is not known which pathogen or combination of pathogens is actually responsible for the disease. Epidemiological studies in the United States as well as in other parts of the world show that the most prevalent enteropathogens associated with neonatal calf diarrhea include, but are not limited to, *Cryptosporidium parvum*, rotavirus, coronavirus and *E. coli.* While in most cases several of these enteropathogens are isolated from outbreaks of the disease, the prevalence of each of the agents is not consistent within a single diseased population or between multiple infected herds. Traditionally, studies found rotavirus to be the most prevalent enteropathogen in diarrheic calves. For example, in a study of diarrheic calves in Great Britain, rotavirus and *Cryptosporidium parvum* were detected in 42 and 23% of the population, respectively. Twenty percent of the calves were infected with more than one pathogen. However, more recent reports indicate *Cryptosporidium parvum* to be the predominant pathogen in enteric bovine infections. In a recent study evaluating *Cryptosporidium parvum* and concurrent infections by other major enteropathogens in neonatal calves, *Cryptosporidium parvum* was the only enteropathogen found in 52.3% of the population, followed by single infections with rotavirus at 42.7%. de la Fuente et al., Preventive Veterinary Medicine 36: 145 - 152 (1998) Concurrent infection with two agents occurred in 21.6% of this study group while infection with three and four pathogens was found in 6% and 0.5%, respectively. The most common mixed infection in this study was a combination of *Cryptosporidium*-rotavirus. There is limited information available on the role of individual enteric pathogens in neonatal calf diarrhea. Furthermore, combined mechanisms of viral, bacterial and protozoal pathogenesis underlying the bovine enteric disease in neonatal animals are even more poorly understood. However, irrespective of the lack of understanding of the mechanism of pathogenesis, infection with more than one pathogen tends to lead to a more severe clinical outcome than infections caused by a single enteropathogen.

At the present time there is no method of treatment that affords adequate protection against neonatal calf diarrhea. There is no single drug or combination of chemotherapeutic agents useful in the treatment of this disease. While vaccines are available which target bovine enteric disease, they have been met with limited success and acceptance. Presently available are vaccines that contain antigens to three enteropathogens found to be associated with the disease, namely rotavirus, coronavirus and *E. coli.* Efficacy of individual components of these commercially available bovine enteric vaccines (rota/corona, *E. coli*) have been shown to protect in experimental challenge models. Despite the availability of such vaccines, under field conditions neonatal diarrhea, calf scours and winter dysentery continue to affect beef, feedlot and cow calf operations. Producers permanently question the efficacy of current enteric vaccines containing *E. coli* K99, rota and coronavirus under field conditions as is reflected by the low usage of the enteric combo vaccines in the US market (only 4% of pregnant animals are vaccinated annually with this product).

More recently, a monovalent experimental vaccine against *Cryptosporidium parvum* has been developed and shown to protect against a *Cryptosporidium parvum* experimental challenge. However, the multiple enteropathogens involved in enteric disease cannot be overcome by treatment with a *Cryptosporidium parvum* vaccine alone. Also, enteropathogenic infection appears to be universal; it is found throughout the world and most vertebrates are susceptible to such infection. Therefore, a need to combat enteropathogenic infection is not limited to the bovine species. Furthermore, enteric disease is difficult to control: it is likely multifactoral; *Cryptosporidium parvum* may be a factor, but heretofore there is no definitive shewing that *Cryptosporidium parvum* indeed enhances enteric disease or that its use in a combination immunogenic, immunological or vaccine composition enhances prevention of enteric disease.

Further, a problem encountered in the preparation and use of combination vaccines is the phenomenon called "efficacy interference" wherein the efficacy of one antigen in the combination is diminished or reduced, believed to be from dominance by another antigen in the combination vaccine: *cf*. Paoletti et al., U.S. Patent No. 5,843,456. This phenomenon has been observed with combination vaccines that employ *E. coli* antigen or antigens; for instance, single or multiple bacterin can interfere with other antigens in combination vaccines.

Thus, it is believed that heretofore the problem of *Cryptosporidium parvum* contributing to enteric infections and symptoms, or the manner in which this problem is herein addressed, e.g., combination compositions including *Cryptosporidium parvum* antigen(s) or epitope(s) of interest with at least one other antigen or epitope of interest from a pathogen that causes enteric infection and/or symptoms and/or recombinant(s) and/or vector(s) and/or plasmid(s) expressing such antigen(s) or epitope(s) of interest and administration of such compositions to pregnant mammals such as pregnant cows and/or newborn or young mammals such as calves within the first month of birth, and addressing any potential issue of efficacy interference, have not been disclosed or suggested.

### OBJECTS AND SUMMARY OF THE INVENTION

An object of the invention enteric immunological or vaccine compositions, especially those which can be used in the veterinary field, for instance for mammals such as bovines, canines, felines or equines or species thereof.

Another object of the invention can be such immunological or vaccine compositions which can be effectively used to immunize newborn and/or young animals, such as to passively, immunize new-born animals, e.g., mammals, for instance, bovines, canines, felines or equines or species thereof; advantageously bovines.

Still another object of the invention can be improved immunological or vaccine compositions against *Cryptosporidium parvum*, for instance particular to be used in the veterinary field, such as for use with mammals, e.g., for canines, felines or equines or species thereof, especially bovines or species thereof.

There is mentioned herein methods for immunizing newborns and/or young animals, such as to passively immunize newborn animals, e.g., mammals, such as canines, felines or equines or species thereof especially bovines or species thereof.

Even further still there is mentioned herein methods for eliciting an immune response against *Cryptosporidium parvum* or enteric pathogens including *Cryptosporidium parvum* or for controlling, preventing and/or treating enteric infections and/or symptoms including *Cryptosporidium parvum;* for instance, comprising administering an inventive composition; as well as methods for preparing such compositions, uses of components of such compositions for formulating such compositions, *inter alia.*

There is provided, in one aspect of the present invention, a combined enteric immunological or vaccine composition comprising:
a first antigen or epitope of interest from *Cryptosporidium parvum* and/or vector that expressed the first antigen or epitope of interest,
and a second antigen or epitope of interest from another enteric pathogen and/or the first vector that expresses the first antigen or epitope of interest also expresses the second antigen or epitope of interest and/or a second vector that expresses the second antigen or epitope of interest ,
and a pharmaceutically acceptable vehicle;
wherein the antigen from the enteric pathogen is selected from the group consisting of the antigens from *E. coli,* rotavirus, coronavirus, and mixtures thereof.
The present invention provides, in another aspect, a composition according to the present invention wherein said composition is used as a bovine, canine, feline or equine combined enteric immunological or vaccine composition.
In another aspect of the present invention, there is provided the use of a composition according to the present invention in the preparation of a medicament for the prevention and/or treatment and/or control of *Cryptosporidium parvum* and/or enteric infections in animals.
In a further aspect of the present invention, there is provided a method for preparing a composition according to the present invention comprising admixing the antigens or epitopes or vectors and a carrier.
In another aspect, there is provided a kit for preparing a composition according to the present invention comprising the antigens, epitopes or vectors each in separate container or containers, optionally packaged together; and further optionally with instructions for admixture and/or administration.
The present invention provides, in another aspect, the use of a composition according to the present invention in the preparation of a medicament for the prevention and/or treatment and/or control of *Cryptosporidium parvum* and/or enteric infections in animals.
Vaccination or immunization against enteric pathogens, such as enteric pathogens including *Cryptosporidium parvum* is greatly and unexpectedly improved by using an immunological or vaccine composition including a combination of at least two *Cryptosporidium parvum* antigens or epitopes thereof and/or vector(s) expressing at least two *Cryptosporidium parvum* antigens or epitopes thereof, e.g., P21 or an epitope thereof and/or a vector expressing P21 or an epitope thereof or Cp23 or an epitope thereof and or/ a vector expressing Cp23 or an epitope thereof and Cp15/60 or an epitope thereof and/or a vector expressing Cp15/60 (for instance, a composition containing at least one epitope of Cp23 and at least one epitope of Cp15/60; and it is noted that the Cp23 antigen or protein can include P21). The combination of both antigens (or epitope(s) of interest and/or vectors expressing the antigens and/or epitope(s)) leads to a synergistic effect with an improved or useful production of an immune response, e.g., antibodies, cellular responses or both, against *Cryptosporidium parvum* and/or enteric infection or pathogens or symptoms such as a very high production of antibodies against *Cryptosporidium parvum*. This also allows for the preparation of efficient immunological or vaccine compositions, useful to protect newborn or young animals or mammals, for instance, canines, felines or equines or species thereof; especially bovines. For instance, compositions containing antigens and/or epitope(s) of interest may be advantageously employed in inoculating dams or pregnant females, e.g., to elicit an immune response that can be passed to the yet born offspring and to new-born or young animals via milk or colostrum during weaning, and, compositions containing vector(s) expressing antigens and/or epitope(s) may advantageously be employed in inoculating males and females of all ages, e.g., such as those that are not pregnant and/or are new-born or young animals, and the inoculation of new-born or young animals can be done alone or advantageously in conjunction with the inoculation of dams or pregnant females, e.g., to allow for immune responses to be generated in the young or new-born animals while they also receive antibodies or other immunological agents via milk or colostrum during nursing.

Combining in an immunological or vaccine composition antigen(s) and/or epitope(s) of interest against *Cryptosporidium parvum* with at least one other antigen or epitope of interest against at least one other enteric pathogen of the animal species (and advantageously a plurality of antigen(s) and/or epitope(s) of interest from a plurality of pathogen(s), e.g., enteric pathogens) can significantly increase protection against enteric pathologies.

An especially advantageous inventive immunological or vaccine composition can be against *Cryptosporidium parvum* and can comprise (i) at least one Cp23 antigen or epitope of interest thereof and/or at least one vector expressing at least one Cp23 antigen or epitope of interest thereof or at least one P21 antigen or epitope of interest thereof and/or at least one vector expressing at least one P21 antigen or epitope of interest thereof and (ii) at least one Cp15/60 antigen or epitope of interest thereof and/or at least one vector expressing at least one Cp 15/60 The composition advantageously further comprises at least one additional antigen or epitope of interest from another enteric pathogen and/or a vector expressing at least one additional antigen (which can be the same vector that expresses the Cp23 or P21 antigen or epitope of interest and/or the Cp15/60 antigen or epitope of interest, e.g., the composition can comprise a vector that co-expresses the Cp23 or P21 antigen or epitope of interest and the Cp15/60 antigen or epitope of interest, and optionally the optional additional antigen or epitope of interest).

Another *Cryptosporidium parvum* antigen is the CP41 antigen described in Mark C. Jenkins et al., Clinical and Diagnostic Laboratory Immunology, Nov. 1999, 6, 6 : 912-920. The immunological or vaccine compositions according to the invention may comprise this antigen or epitope of interest thereof and/or a vector expressing said antigen or epitope thereof, possibly and preferably in association with at least one other *Cryptosporidium parvum* as described herein such as Cp23, P21 and Cp 15/60, e.g. in combination with Cp23 or P21 and/or Cp15/60. For expression of this antigen, one may add a start codon upstream the nucleotide sequence appearing on Figure 2 of this publication, and a stop codon downstream this sequence.

An efficient immunological or vaccine composition against enteritis is also produced by using only one of: the Cp23 or an epitope thereof or a vector expressing the antigen or epitope, or P21 or an epitope thereof or a vector expressing the antigen or epitope, or Cp15/60 or an epitope thereof or a vector expressing the antigen or epitope thereof, or CP41 or an epitope thereof or a vector expressing the antigen or epitope, as a *Cryptosporidium parvum* antigen or epitope of interest, advantageously in combination with at least one other *Cryptosporidium parvum* antigen or epitope of interest or vector expressing such an antigen or epitope of interest; and, this composition can further comprise at least one additional antigen or epitope of interest from another enteric pathogen and/or a vector expressing the at least one additional antigen (and this vector can co-express antigen(s) and/or epitope(s)).

There is mentioned herein methods for eliciting an immunological or protective (vaccine) response against or for controlling, preventing and/or treating enteric pathogens or enteric infections or enteric symptoms, including *Cryptosporidium parvum*; for instance, comprising administering an inventive composition. -

An inventive composition can be administered to a pregnant mammal, such as a heifer or a cow (hereinafter called cow), dog, cat, or horse during the gestation period; for instance, once or twice during the typical gestation period (for a cow, typically a 9 month or 170 day gestation period), such as a first administration about 1 to about 2.5 or about 3 months before calving and a second or sole administration close to calving, e.g., in the last 3 weeks before calving, preferably about 3 to about 15 days before calving. In this way, the female can transfer passive immunity to the newborn, e.g., calves after birth via milk or colostrum. Advantageously, compositions comprising antigen(s) and/or epitope(s) of interest (as opposed to compositions comprising vector(s), recombinant(s) and/or DNA plasmid(s)) are administered to pregnant mammals as eliciting an antibody response is desired. And, in contrast, such compositions that comprise vector(s), recombinant(s) and/or DNA plasmid(s) that express the antigen(s) and/or epitope(s) of interest *in vivo* are advantageously administered to a newborn or very young mammal (e.g., a mammal that is susceptible to enteric disease, such as a bovine during about its first month of life and other mammals during analogous periods in their life), as a cellular and/or antibody response can be useful to prevent, treat, and/or control enteric conditions, infections or symptoms in such newborn and/or very young animals. The newborn and/or very young animals can receive a booster of an antigenic and/or epitopic and/or vector/recombinant/DNA plasmid composition during the period of susceptibility; and, its mother, optionally and advantageously, can also have been vaccinated during pregnancy, as herein described, such that the newborn and/or very young animal can be receiving an immunological response by way of the administration directly to it and passively.

A particular inventive composition can comprise one or more *E. coli* antigens (e.g., inactivated *E. coli* bearing pili, such as, K99, Y, 31A, and/or F41 and/or these pili in subunit form or recombinantly expressed *in vivo*) and/or one or more rotavirus antigens (e.g., advantageously inactivated rotavirus), and/or one or more coronavirus antigen (e.g., bovine coronavirus antigen, advantageously such as inactivated coronavirus), in combination with one or more *Cryptosporidium parvum* antigens, such as P21 and/or Cp23 and/or Cp15/60. (And, as mentioned previously, one or more of these antigens can be an epitope of interest contained within the antigen; and, one or more of these antigens or epitopes of interest can be expressed *in vivo* by a recombinant or a plasmid.)

Thus, a particular inventive composition can comprise (i) one or more *Cryptosporidium parvum* antigens, such as P21 and/or Cp23 and/or Cp15/60 and/or CP41 and advantageously P21 and/or Cp23 and Cp15/60, and (ii) at least one *E. coli* antigen (e.g., at least one or all of of K99, Y, 31A, F41 and/or other pili borne by inactivated *E. coli* or as subunits or as expressed *in vivo*; K99 and/or F41 are preferably present and Y and/or 31A are advantageously also present), and/or coronavirus and/or rotavirus antigen; such as one or more *C. parvum* antigens, such as P21 and/or Cp23 and/or Cp15/60 and/or CP41 and advantageously P21 and/or Cp23 and Cp15/60 and one or more rotavirus antigen such as inactivated rotavirus, or one or more *C. parvum* antigens, such as P21 and/or Cp23 and/or Cp15/60 and/or CP41 and advantageously P21 and/or Cp23 and Cp15/60 and one or more coronavirus antigen such as inactivated coronavirus, e.g., inactivated bovine coronavirus, or one or more *C. parvum* antigens, such as P21 and/or Cp23 and/or Cp15/60 and/or CP41 and advantageously P21 and/or Cp23 and Cp15/60 and one or more *E. coli* antigen such as K99, Y, 31A, F41 and/or other pili borne by inactivated *E. coli* or as subunits or as expressed *in vivo,* e.g., a combination of K99, Y, 31A and/or F41. An exemplary *E. coli* antigen useful in the invention can be pili as *E. coli* pili can avoid efficacy interference. An exemplary composition can comprise one or more *C. parvum* antigens, such as P21 and/or Cp23 and/or Cp15/60 and/or CP41 and advantageously P21 and/or Cp23 and Cp15/60 and at least one *E. coli* antigen, and at least one coronavirus antigen, and at least one rotavirus antigen, e.g., P21 and/or Cp23 and/or Cp15/60 and/or CP41 and advantageously P21 and/or Cp23 and Cp15/60 and inactivated rotavirus, and inactivated coronavirus, and at least one *E coli* antigen, advantageously pili or preferably at least one or more of K99, Y, 31A, and F41, or a combination of K99, Y, 31A and F41. (And, as mentioned previously, one or more of these antigens can be an epitope of interest contained within the antigen; and, one or more of these antigens or epitopes of interest can be expressed *in vivo* by a recombinant or a' plasmid.) In regard to potential efficacy interference by single or multiple bacterin, the inventors have found that by increasing the amount of other antigens present in a combination vaccine, any potential efficacy interference is avoided; and, that the use of pili as an *E. coli* antigen also avoids efficacy interference.

In these inventive compositions, a single dose can have the *E. coli* antigen (or each *E. coli* antigen, in the case of multiple *E. coli* antigens) present in an amount usually found in vaccines against enteric pathogens such as an amount to obtain a serum titre in guinea pigs of at least 0.9 log 10; the rotavirus antigen can be present in an typically found in vaccines against enteric pathogens, such as an amount to obtain a serum titre in guinea pigs of at least 2.0 log 10, and the coranovirus antigen can be present in an amount typically found in vaccines against enteric pathogens such as an amount to obtain a serum titre in guinea pigs of at least 1.5 log 10; and, the inventive compositions can include an adjuvant, such as aluminum hydroxide, which can be present in a single dose in an amount typically found in vaccines such as preferably an amount of about 0.7 to about 0.9 mg.

Accordingly, in an aspect the invention provides combined enteric immunological, immunogenic or vaccine composition comprising a first antigen or epitope of interest from *Cryptosporidium parvum* and/or a first vector that expresses the first antigen or epitope of interest, and a second antigen or epitope of interest from another enteric pathogen and/or the first vector that expresses the first antigen or epitope of interest also expresses the second antigen or epitope of interest and/or a second vector that expresses the second antigen or epitope of interest, and a pharmaceutically acceptable vehicle.

The composition can comprise antigen which can be from *Cryptosporidium parvum* and an antigen from another enteric pathogen. The composition can comprise an antigen from *Cryptosporidium* and an antigen from another enteric pathogen of a bovine species; or of a canine species; or of a feline species; or of an equine species. The antigen from the enteric pathogen can be chosen from the group consisting of the antigens from *E. coli*, rotavirus, coronavirus, *Clostridium spp.* and mixtures thereof. The enteric pathogen can be *E. coli.* The antigen from *E. coli* can be selected from the group consisting of *E. coli* bearing K99 antigen, *E. coli.* bearing F41 antigen, *E. coli* bearing Y antigen, *E. coli* bearing 31A antigen. K99 antigen, F41 antigen, Y antigen, 31A antigen, and mixtures thereof.

There is mentioned herein a composition wherein the enteric pathogen can comprise bovine coronavirus; and/or bovine rotavirus and/or *Clostridium perfringens.* The antigen of the enteric pathogen can comprise *Clostridium perfringens* type C and D toxoids. There is mentioned herein a composition wherein the enteric pathogen can comprise *E. coli,* bovine rotavirus, bovine coronavirus and *Clostridium perfringens* or *E. coli,* bovine rotavirus, bovine coronavirus.

Yet further, there is mentioned herein a composition wherein the antigen of the enteric pathogen comprises *E. coli* antigens selected from the group consisting of *E. coli* bearing K99 antigen, *E. coli.* bearing F41 antigen, *E. coli* bearing Y antigen, *E. coli* bearing 31A antigen, K99 antigen, F41 antigen, Y antigen, 31A antigen, and mixtures thereof; inactivated bovine coronavirus; inactivated bovine rotavirus and *Clostridium perfringens* type C and D toxoids; or *E. coli* antigens selected from the group consisting of *E. coli* bearing K99 antigen, *E. coli.* bearing F41 antigen, *E. coli* bearing Y antigen, *E. coli* bearing 31A antigen, K99 antigen, F41 antigen, Y antigen, 31A antigen and mixtures thereof; inactivated bovine coronavirus; and inactivated bovine rotavirus.

The inventive composition advantageously can comprise sub-unit *Cryptosporidium parvum* antigens selected from the group consisting of P21, Cp23, Cp15/60, CP41 and mixtures thereof, such as Cp23 and Cp15/60 or P21 and Cp15/60.

In the inventive compositions associating antigens from *Cryptosporidium parvum* and at least one other enteric pathogen, the *Cryptosporidium parvum* antigen may also comprise or be constituted by, inactivated or live attenuated oocysts, or sub-units obteined from oocysts.

Inventive compositions can include an adjuvant such as saponin or aluminum hydroxyde; and, inventive compositions can be in the form of an oil-in-water emulsion.

There is mentioned herein an immunological, immunogenic or vaccine composition against *Cryptosporidium parvum,* which comprises a first antigen comprising a P21 or Cp23 antigen or an epitope thereof or a first vector that expresses the first antigen and a second antigen comprising Cp15/60 antigen or epitope thereof or the first vector wherein the first vector expresses both the first and second antigens or a second vector that expresses the second antigen, and a pharmaceutically acceptable vehicle. The composition can comprise Cp23 and Cp15/60 antigens which are in the form of separate fusion proteins. The composition can comprise a vector expressing Cp23 and Cp15/60. The composition can comprise a first recombinant vector expressing Cp23 and a second recombinant vector expressing Cp15/60. And, the composition can comprise P21 and Cp15/60. These compositions can further comprise an adjuvant.

Still further, there is mentioned herein an immunological, immunogenic or vaccine composition against *Cryptosporidium parvum*, which comprises a first antigen comprising a P21 or Cp23 or Cp15/60 or CP41 antigen or an epitope thereof or a first vector that expresses the first antigen and a second antigen comprising a second antigen or epitope thereof from *Cryptosporidium parvum* or the first vector wherein the first vector expresses both the first and second antigens or a second vector that expresses the second antigen, wherein the first and second antigens are different from each other, and a pharmaceutically acceptable vehicle.

There is also mentioned herein a method of bovine immunization of a newborn calf against enteric disease comprising administering an inventive composition to a pregnant female calf before delivering, so that the newborn calf receives maternal antibodies against *Cryptosporidium parvum* through colostrum and/or milk. The method can further comprise the feeding to the newborn calf colostrum and/or milk from cow(s) which has (have) been administered the composition during pregnancy. The method can comprise administering the composition to the new-born calf. The composition administered to the pregnant female can comprise antigens or epitopes thereof and the composition administered to the calf can comprise vectors. Thus, there is also mentioned herein a method of active immunization of adult and newborn calves, comprising administering to the calves an inventive composition.

There is also mentioned herein a method of bovine immunization of a newborn calf, comprising feeding to the new-born calf colostrum and/or milk from cows which have been administered the composition during pregnancy. Similarly, in a broader sense, there is mentioned herein a method of immunization of a new-born mammal comprising feeding to the new-born colostrum and/milk from a female mammal which has been administered the composition during pregnancy; and, the mammal is advantageously, a bovine, a feline, a canine, or an equine. Still further, the invention can encompass a method for preparing an inventive composition comprising admixing the antigens or epitopes or vectors and the carrier.

And, the invention can include a kit for preparing an inventive composition comprising the antigens, epitopes or vectors, each in separate container or containers (some antigens, epitopes or vectors may be together in one container, such as the *Cryptosporidium parvum* antigens, epitopes or vectors may be together in one, container, and the other antigens, epitopes or vectors in one or more other containers, or the carrier, diluent and/or adjuvant may be in separate containers), optionally packaged together, and further optionally with instructions for admixture and/or administration.

The term "comprising" in this disclosure can mean "including" or can have the meaning commonly given to the term "comprising" in U.S. Patent Law.

Other aspects of the invention are described in or are obvious from (and within the ambit of the invention) the following disclosure.

### BRIEF DESCRIPTION OF FIGURES

The following Detailed Description, given by way of example, and not intended to limit the invention to specific embodiments described, may be understood in conjunction with the accompanying Figures, incorporated herein by reference, in which:
Figure 1 shows a physical and restriction map of plasmid pJCA155;
Figure 2 shows a physical and restriction map of plasmid pJCA156;
Figure 3 shows a physical and restriction map of plasmid pJCA157;
Figure 4 shows a physical and restriction map of plasmid pJCA158;
Figure 5 shows a physical and restriction map of plasmid pJCA159;
Figure 6 shows a physical and restriction map of plasmid pJCA160;
Figure 7 shows comparative oocysts count in feces in calves challenged with either C. parvum, or bovine rotavirus, or both, or non challenged (example 12);
Figure 8 shows comparative rotavirus excretion in feces in calves according to example 12;
Figure 9 shows comparative animal general condition for calves according to example 12;
Figure 10 shows comparative animal dehydration status in calves according to example 12;
Figure 11 shows comparative count of liquid feces for calves according to example 12;
Figure 12 shows comparative anorexia status for calves according to example 12; and
Figure 13 shows comparative rectal temperature evolution in calves according to example 12.

### DETAILED DESCRIPTION

An aspect of the invention is thus a combined enteric immunological, immunogenic or vaccine composition comprising at least one an antigen or epitope of interest from at least one *Cryptosporidium spp.,* preferably including *Cryptosporidium parvum*, and at least one antigen from at least one other enteric pathogen, advantageously a pathogen infecting the animal species to be protected, such as canine, feline, equine or bovine species and more advantageously bovine species; and/or a vector or vectors and/or a recombinant or recombinants and/or a plasmid or plasmids that expresses the *Cryptosporidium spp* antigen or epitope of interest and/or at least one of the antigen(s) or epitope(s) of interest of the other enteric pathogen; and a pharmaceutically acceptable vehicle. Universal immunological, immunogenic or vaccine compositions are also envisioned as enteric pathogens are often infecting several (more than one) animal species.

An immunological composition elicits an immunological response - local or systemic. An immunogenic composition likewise elicits a local or systemic immunological response. A vaccine composition elicits a local or systemic protective response. Accordingly, the terms "immunological composition" and "immunogenic composition" include a "vaccine composition" (as the two former terms can be protective compositions).

*Cryptosporidium parvum* antigens which can be used in this invention comprise preferably :
(1) A protein of 148 amino acids called Cp15/60 (*See, e.g.,* U.S. Patent No. 5,591,434. This protein is represented in US-A-5,591,434 in SEQ ID NO:2 with 10 further amino acids at the 5' end, upstream the methionine (Met). It is within the scope of the present invention to use an antigen comprising or consisting essentially of the 148 amino acid sequence of Cp15/60 or of a longer amino acid sequence including these 148 amino acids, e.g. the whole sequence represented in SEQ ID NO:2 in US-A-5,591,434 or any polypeptide comprising a fragment of the 148 or 158 amino acid sequences that comprises an epitope thereof, advantageously a protection-eliciting epitope or an epitope that has the immumogenicity of the full length sequence.) and/or
(2) Cp23 and/or P21. (Cp23 is an antigen of about 23 kDa; *see* Perryman et al., Molec Biochem Parasitol 80:137-147 (1996); WO-A-9807320 and L. E. Perryman et al., Vaccine 17 (1999) 2142-2149. The major part of this protein (187 amino acids) is herein termed P21 and has an amino acid sequence homologous to the amino acid sequence of protein C7 which is disclosed as SEQ ID NO. 12 in WO-A-98 07320 To be expressed, one or two or more amino acids can be added at the end of P21, such as, Met-, or Met-Gly- or similar amino acids. It is within the scope of the present invention to use an antigen comprising or consisting essentially of or consisting of the 187 amino acid sequence or a longer amino acid sequence, or a polypeptide comprising a fragment of the 187 amino acid sequence that comprises an epitope thereof, advantageously a protection-eliciting epitope or an epitope that has the immunogenicity of the full length sequence. The whole amino acid sequence of Cp23 and the corresponding nucleotide sequence is easily obtainable. The P21 protein represents the major part and the C-terminal end of Cp23. The P21 nucleotide sequence may be used as a probe to screen a DNA library, e.g. a library as disclosed in Example 1. This methodology is well known to the one skilled in the art. On the basis of the molecular weight of Cp23, it can be asserted that about 25-35 amino acids are missing at the N-terminal end of P21 to have the complete Cp23 amino acid sequence. This information gives those skilled in the art the means to easily find the start codon and thus the 5' end of the Cp23 nucleotide sequence and the N-terminal amino acid sequence.

The antigens or epitopes of interest can be used individually or in combination in compositions of the invention, e.g., an inventive composition can include (1) or (2) or both (1) and (2).

Another possible antigen is the CP41 antigen as disclosed supra.

According to the preferred embodiment, these antigens or epitopes of interest are incorporated into the composition as proteins or sub-unit antigens. They can be produced by chemical synthesis or by expression *in vitro.* The examples describe how to obtain the sequences encoding Cp15/60 and P21 and how to construct vectors expressing them. These sequences can be cloned into suitable cloning or expression vectors. These vectors are then used to transfect suitable host cells. The antigens encoded by the nucleotide sequence which is inserted into the vector, e.g. Cp23 and/or P21 and/or Cp15/60, are produced by growing the host cells transformed by the expression vectors under conditions whereby the antigen is produced. This methodology is well known to the one skilled in the art. Host cells may be either procaryotic or eucaryotic, e.g. *Escherichia coli (E. coli*), yeasts such as *Saccharomyces cerevisiae*, animal cells, in particular animal cell lines. The one skilled in the art knows the vectors which can be used with a given host cell. The vectors may be chosen such that a fusion protein is produced which can be used then to easily recover the antigen.

Furthermore, with respect to sequences, nucleic acid sequences useful for expressing the *C. parvum* antigen or epitope of interest can include nucleic acid sequences that are capable of hybridizing under high stringency conditions or those having a high homology with nucleic acid molecules employed in the invention (e.g., nucleic acid molecules in documents mentioned herein); and, "hybridizing under high stringency conditions" can be synonymous with "stringent hybridization conditions", a term which is well known in the art; see, for example, Sambrook et al., "Molecular Cloning, A Laboratory Manual" second ed., CSH Press, Cold Spring Harbor, 1989; "Nucleic Acid Hybridisation, A Practical Approach", Hames and Higgins eds., IRL Press, Oxford, 1985.

With respect to nucleic acid molecules and polypeptides which can be used in the practice of the invention, the nucleic acid molecules and polypeptides advantageously have at least about 75% or greater homology or identity, advantageously 80% or greater homology or identity, more advantageously 85% or greater homology or identity, such as at least about 85% or about 86% or about 87% or about 88% or about 89% homology or identity, for instance at least about 90% or homology or identity or greater, such as at least about 91%, or about 92%, or about 93%, or about 94% identity or homology, more advantageously at least about 95% to 99% homology or identity or greater, such as at least about 95% homology or identity or greater e.g., at least about 96%, or about 97%, or about 98%, or about 99%, or even about 100% identity or homology, or from about 75%, advantageously from about 85% to about 100% or from about 90% to about 99% or about 100% or from about 95% to about 99% or about 100% identity or homology, with respect to sequences set forth in herein cited documents (including subsequences thereof discussed herein); and thus, the invention comprehends a vector encoding an epitope or epitopic region of a *C. parvum* isolate or a composition comprising such an epitope, compositions comprising an epitope or epitopic region of a *C. parvum* isolate, and methods for making and using such vectors and compositions, e.g., the invention also comprehends that these nucleic acid molecules and polypeptides can be used in the same fashion as the herein mentioned nucleic acid molecules, fragments thereof and polypeptides.

Nucleotide sequence homology can be determined using the "Align" program of Myers and Miller, ("Optimal Alignments in Linear Space", CABIOS *4*, 11-17, 1988, incorporated herein by reference) and available at NCBL Alternatively or additionally, the term "homology" or "identity", for instance, with respect to a nucleotide or amino acid sequence, can indicate a quantitative measure of homology between two sequences. The percent sequence homology can be calculated as (N*_{ref}* - N*_{dif}*)*100/N*_{ref}*, wherein N*_{dif}* is the total number of non-identical residues in the two sequences when aligned and wherein N*_{ref}* is the number of residues in one of the sequences. Hence, the DNA sequence AGTCAGTC will have a sequence similarity of 75% with the sequence AATCAATC (N*_{ref}* = 8; N*_{dif}*=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman, 1983 PNAS USA 80:726), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. RNA sequences within the scope of the invention can be derived from DNA sequences, by thymidine (T) in the DNA sequence being considered equal to uracil (U) in RNA sequences.

Additionally or alternatively, amino acid sequence similarity or identity or homology can be determined using the BlastP program (Altschul et al., NncL Acids Res. 25, 3389-3402),
and available at NCBI (used in determining sequence homology, as shown in Appendix I; *see also* the Examples). The following references
also provide algorithms for comparing the relative identity or homology of amino acid residues of two proteins, and additionally or alternatively with respect to the foregoing, the teachings in these references can be used for determining percent homology or identity: Needleman SB and Wunsch CD, "A general method applicable to the search for similarities in the amino acid sequences of two proteins," J. Mol. Biol. 48:444-453 (1970); Smith TF and Waterman MS, "Comparison of Bio-sequences," Advances in Applied Mathematics 2:482-489 (1981); Smith TF, Waterman MS and Sadler JR, "Statistical characterization of nucleic acid sequence functional domains," Nucleic Acids Res., 11:2205-2220 (1983); Feng DF and Dolittle RF, "Progressive sequence alignment as a prerequisite to correct phylogenetic trees," J. of Molec. Evol., 25:351-360 (1987); Higgins DG and Sharp PM, "Fast and sensitive multiple sequence alignment on a microcomputer," CABIOS, 5: 151-153 (1989); Thompson JD, Higgins DG and Gibson TJ, "ClusterW: improving the sensitivity of progressive multiple sequence alignment through sequence weighing, positions-specific gap penalties and weight matrix choice, Nucleic Acid Res., 22:4673-480 (1994); and, Devereux J, Haeberlie P and Smithies O, "A comprehensive set of sequence analysis program for the VAX." Nucl. Acids Res., 12: 387-395 (1984).

Furthermore, as to nucleic acid molecules used in this invention (e.g., as in herein cited documents), the invention comprehends the use of codon equivalent nucleic acid molecules. For instance, if the invention comprehends "X" protein (e.g., P21 and/or Cp23 and/or Cp15/60 and/or CP41) having amino acid sequence "A" and encoded by nucleic acid molecule "N", the invention comprehends nucleic acid molecules that also encode protein X via one or more different codons than in nucleic acid molecule N.

The antigen or epitope of interest used in the practice of the invention can be obtained from the particular pathogen(s), e.g., *C. parvum, E. coli,* rotovirus, coronavirus, and the like or can be obtained from *in vitro* and/or *in vivo* recombinant expression of gene(s) or portions thereof. Methods for making and/or using vectors (or recombinants) for expression can be by or analogous to the methods disclosed in: U.S. Patent Nos. 4,603,112, 4,769,330, 5,174,993, 5,505,941, 5,338,683, 5,494,807, 4,722,848, 5,942,235, PCT publications WO 94/16716, WO 96/39491. Paoletti. "Applications of pox virus vectors to vaccination: An update," PNAS USA 93:11349-11353, October 1996, Moss, "Genetically engineered poxviruses for recombinant gene expression, vaccination, and safety," PNAS USA 93:11341-11348, October 1996, Smith et al., U.S. Patent No. 4.745,051 (recombinant baculovirus), Richardson, C.D. (Editor), Methods in Molecular Biology 39, "Baculovirus Expression Protocols" (1995 Humana Press Inc.), Smith et al., "Production of Huma Beta Interferon in Insect Cells Infected with a Baculovirus Expression Vector," Molecular and Cellular Biology, Dec., 1983, Vol. 3, No. 12, p. 2156-2165; Pennock et al., "Strong and Regulated Expression of Escherichia coli B-Galactosidase in Infect Cells with a Baculovirus vector," Molecular and Cellular Biology Mar. 1984, Vol. 4, No. 3, p. 399-406; EPA 0 370 573, EP Patent publication No. 265785, U.S. Patent No. 4,769,331 (recombinant herpesvirus), Roizman, "The function of herpes simplex virus genes: A primer for genetic engineering of novel vectors," PNAS USA 93:11307-11312, October 1996, Andreansky et al., "The application of genetically engineered herpes simplex viruses to the treatment of experimental brain tumors," PNAS USA 93:11313-11318, October 1996, Robertson et al. "Epstein-Barr virus vectors for gene delivery to B lymphocytes," PNAS USA 93:11334-11340, October 1996, Frolov et al., "Alphavirus-based expression vectors: Strategies and applications," PNAS USA 93:11371-11377, October 1996, Kitson et al., J. Virol. 65, 3068-3075, 1991; U.S. Patent Nos. 5,591,439, 5,552,143, 6,156,567 and 6,090,393 recombinant adenovirus), Grunhaus et al., 1992, "Adenovirus as cloning vectors," Seminars in Virology (Vol. 3) p. 237-52, 1993, Ballay et al. EMBO Journal, vol. 4, p. 3861-65, Graham, Tibtech 8, 85-87, April, 1990, Prevec et al., J. Gen Virol. 70, 429-434, PCT WO91/11525, Felgner et al. (1994), J. Biol. Chem. 269, 2550-2561, Science, 259:1745-49, 1993 and McClements et al., "Immunization with DNA vaccines encoding glycoprotein D or glycoprotein B, alone or in combination, induces protective immunity in animal models of herpes simplex virus-2 disease," PNAS USA 93:11414-11420, October 1996, and U.S. Patents Nos 5,591,639, 5,589,466, and 5,580,859 relating to DNA expression vectors, *inter alia*. See also WO 98/33510; Ju et al., Diabetologia, 41:736-739, 1998 (lentiviral expression system); Sanford et al., U:S. Patent No. 4,945,050; Fischbach et al. (Intracel), WO 90/01543; Robinson et al., seminars in IMMUNOLOGY, voL 9, pp.271-283 (1997) (DNA vector systems); Szoka et al., U.S. Patent No. 4,394,448 (method of inserting DNA into living cells); McCormick et al., U.S. Patent No. 5,677,178 (use of cytopathic viruses); U.S. Patent No. 5,928,913 (vectors for gene delivery), and Tartaglia et al. U.S. Patent No. 5,990,091 (vectors having enhanced expression), as well as other documents cited herein. A viral vector, for instance, selected from herpes viruses, adenoviruses, poxviruses, especially vaccinia virus, avipox virus, canarypox virus, as well as DNA vectors (DNA plasmids) are advantageously employed in the practice of the invention, especially for *in vivo* expression (whereas bacterial and yeast systems are advantageously employed for *in vitro* expression).

If the host-vector combination leads to the production of antigen without excretion, for the convenience of their production, and their recovering, these antigens are preferably under the form of fusion proteins (e.g., a HIS tag). In other words, the antigen can comprise the antigen *per se* and foreign amino acids.

Techniques for protein purification and/or isolation from this disclosure and documents cited herein, *inter alia,* and thus within the ambit of the skilled artisan, can be used, without undue experimentation, to purify and/or isolate recombinant or vector expression products and/or antigen(s), in the practice of the invention, and such techniques, in general, can include: precipitation by taking advantage of the solubility of the protein of interest at varying salt concentrations, precipitation with organic solvents, polymers and other materials, affinity precipitation and selective denaturation; column chromatography, including high performance liquid chromatography (HPLC), ion-exchange, affinity, immunoaffinity or dye-ligand chromatography; immunoprecipitation and the use of gel filtration, electrophoretic methods, ultrafiltration and isoelectric focusing, *inter alia.*

As mentioned herein, according to another aspect, the invention comprehends that the antigens and/or epitopes of interest are not incorporated as subunits in the composition, but rather that they are expressed *in vivo;* e.g., the invention comprehends that the composition comprises recombinant vector(s) expressing the antigens *in vivo* when administered to the animal. The vector can comprise a DNA vector plasmid, a herpesvirus, an adenovirus, a poxvirus, including a vaccinia virus, an avipox virus, a canarypox virus, and a swinepox virus, and the like. The vector-based compositions can comprise a vector that contains and expresses a nucleotide sequence of the antigen to be expressed, e.g., Cp15/60 and/or Cp23 for *Cryptosporidium parvum.*

The word plasmid is intended to include any DNA transcription unit in the form of a polynucleotide sequence comprising the sequence to be expressed. Advantageously, the plasmid includes elements necessary for its expression; for instance, expression *in vivo*. The circular plasmid form, supercoiled or otherwise, is advantageous; and, the linear form is also included within the scope of the invention. The plasmid can be either naked plasmid or plasmid formulated, for example, inside lipids or liposomes, e.g., cationic liposomes (*see*, *e.g.,* WO-A-90 11082; WO-A-92 19183; WO-A-96 21797; WO-A-95 20660). The plasmid immunological or vaccine composition can be administered by way of a gene gun, or intramuscularly, or nasally, or by any other means that allows for expression *in vivo*, and advantageously an immunological or protective response.

Applications have been filed which involve DNA and/or vector vaccines or immunogenic or immunological compositions for felines, canines, bovines, and equines, and inventive compositions can include DNA and/or vector vaccines or immunogenic or immunological compositions from these applications and/or inventive compositions can be prepared and/or formulated and/or administered in a fashion analogous to the compositions of these applications.

Compositions for use in the invention can be prepared in accordance with standard techniques well known to those skilled in the veterinary or pharmaceutical or medical arts. Such compositions can be administered in dosages and by techniques well known to those skilled in the veterinary arts taking into consideration such factors as the age, sex, weight, condition and particular treatment of the animal, and the route of administration. The components of the inventive compositions can be administered alone, or can be co-administered or sequentially administered with other compositions (e.g., the *C. parvum* antigen(s) and/or epitope(s) can be administered alone, and followed by the administration sequentially of antigen(s) and/or epitope(s) of other enteric pathogens, or compositions comprising a enteric antigen(s) or epitope(s) can include vectors or recombinants or plasmids that also express enteric antigen(s) or epitope(s) of the same or different pathogens) or with other prophylactic or therapeutic compositions (e.g., other immunogenic, immunological or vaccine compositions). Thus, the invention provides multivalent or "cocktail" or combination compositions and methods employing them. The ingredients and manner (sequential, e.g., as part of a prime-boost regimen, or as part of a booster program wherein immunogenic, immunological or vaccine composition is administered periodically during the life of the animal such as an annual, seasonal, biannual and the like booster program; or coadministration) of administration, as well as dosages, can be determined, taking into consideration such factors as the age, sex, weight, condition and particular treatment of the animal e.g., cow, and, the route of administration, In this regard, reference is made to U.S. Patent No. 5,843,456, and directed to rabies compositions and combination compositions and uses thereof.

Compositions of the invention may be used for parenteral or mucosal administration, preferably by intradermal, subcutaneous or intramuscular routes. When mucosal administration is used, it is possible to use oral, nasal, or vaginal routes.

In such compositions, the vector(s), or antigen(s) or epitope(s) of interest(s) may be in admixture with a suitable carrier, diluent, or excipient such as sterile water, physiological saline, glucose or the like. The compositions can also be lyophilized. The compositions can contain auxiliary substances such as pH buffering agents, adjuvants, preservatives, polymer excipients used for mucosal routes, and the like, depending upon the route of administration and the preparation desired.

Standard texts, such as "REMINGTON'S PHARMACEUTICAL SCIENCE", 17th edition, 1985, may be consulted to prepare suitable preparations, without undue experimentation. Suitable dosages can also be based upon the text herein and documents cited herein.

Adjuvants are substances that enhance the immune response to antigens. Adjuvants, can include aluminum hydroxide and aluminum phosphate, saponins e.g., Quil A, mineral oil emulsions, pluronic polymers with mineral or metabolizable oil emulsion, the water-in-oil adjuvant, the oil-in-water adjuvant, synthetic polymers (e.g.,homo- and copolymers of lactic and glycolic acid, which have been used to produce microspheres that encapsulate antigens, see Eldridge et al., MoL Immunol. 28:287-294 (1993), e.g., biodegradable microspheres), nonionic block copolymers, low molecular weight copolymers in oil-based emulsions (see Hunter et aL, The Theory and Practical Application of Adjuvants (Ed. Stewart-Tull, D.E.S.). John Wiley and Sons, NY, pp51-94 (1995)), high molecular weight copolymers in aqueous formulations (Todd et aL, Vaccine 15:564-570 (1997)), cytokines such as IL-2 and IL-12 (*see, e.g.,* U.S. Patent No. 5,334,379), and GM-CSF (granulocyte macrophage-calony stimulating factor; *see, generally,* U.S. Patents Nos. 4,999,291 and 5,461,663, *see also* Clark et al., Science 1987, 230:1229; Grant et al., Drugs, 1992, 53:516), advantageously GM-CSF from the animal species to be vaccinated, *inter alia*. Certain adjuvants can be expressed *in vivo* with antigen(s) and/or epitope(s); e.g., cytokines, GM-CSF (*see, e*.*g*., C. R. Maliszewski et al. Molec Immunol 25(9):843-50 (1988); S.R. Leong, Vet Immunol and Immunopath 21:261-78 (1989) concerning bovine GM-CSF. A plasmid encoding GM-CSF can be modified to contain and express DNA encoding an antigen from a bovine pathogen for use according to the instant invention and/or an epitope thereof optionally also with DNA encoding an antigen and/or epitope of another bovine pathogen, or can be used in conjunction with such a plasmid).

A further instance of an adjuvant is a compound chosen from the polymers of acrylic or methacrylic acid and the copolymers of maleic anhydride and alkenyl derivative. Advantageous adjuvant compounds are the polymers of acrylic or methacrylic acid which are cross-linked, especially with polyalkenyl ethers of sugars or polyalcohols. These compounds are known by the term carbomer (Phameuropa Vol. 8, No. 2, June 1996). Persons skilled in the art can also refer to U.S. Patent No. 2,909,462 which describes such acrylic polymers cross-linked with a polyhydroxylated compound having at least 3 hydroxyl groups, preferably not more than 8, the hydrogen atoms of at least three hydroxyls being replaced by unsaturated aliphatic radicals having at least 2 carbon atoms. The preferred radicals are those containing from 2 to 4 carbon atoms, e.g. vinyls, allyls and other ethylenically unsaturated groups. The unsaturated radicals may themselves contain other substituents, such as methyl. The products sold under the name Carbopol® (BF Goodrich, Ohio, USA) are particularly appropriate. They are cross-linked with an allyl sucrose or with allyl pentaerythritol. Among then, there may be mentioned Carbopol® 974P, 934P and 971P. Among the copolymers of maleic anhydride and alkenyl derivative, the copolymers EMA® (Monsanto) which are copolymers of maleic anhydride and ethylene, linear or cross-linked, for example cross-linked with divinyl ether, are preferred. Reference may be made to J. Fields et al., Nature, 186 : 778-780, 4 June 1960.

From the point of view of their structure, the polymers of acrylic or methacrylic acid and the copolymers EMA® are preferably formed of basic units of the following formula: 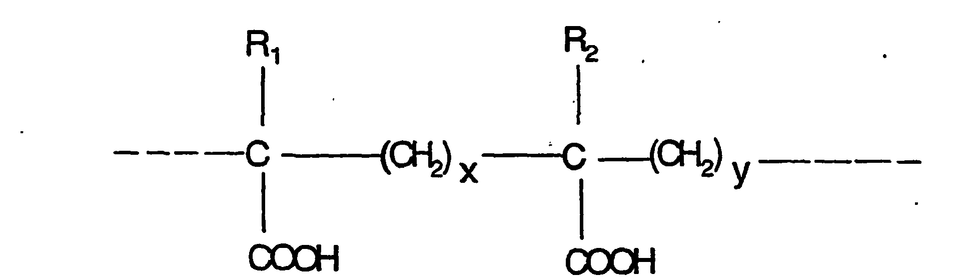 in which:
R₁ and R₂, which are identical or different, represent H or CH_{3:}
x = 0 or 1, preferably x = 1; and
y = 1 or 2, with x + y = 2.

For the copolymers EMA®, x = 0 and y = 2. For the carbomers, x = y =1.

The dissolution of these polymers in water leads to an acid solution that will be neutralized, preferably to physiological pH, in order to give the adjuvant solution into which the immunogenic, immunological or vaccine composition itself will be incorporated. The carboxyl groups of the polymer are then partly in COO⁻ form.

Preferably, a solution of adjuvant according to the invention, especially of carbomer, is prepared in distilled water, preferably in the presence of sodium chloride, the solution obtained being at acidic pH. This stock solution is diluted by adding it to the desired quantity (for obtaining the desired final concentration), or a substantial part thereof, of water charged with NaCI, preferably physiological saline (NaCI 9 g/l) all at once in several portions with concomitant or subsequent neutralization (pH 7.3 to 7.4), preferably with NaOH. This solution at physiological pH will be used as it is for mixing with the vaccine, which may be especially stored in freeze-dried, liquid or frozen form.

The polymer concentration in the final vaccine composition can be 0.01% to 2% w/v, e.g., 0.06 to 1% w/v, such as 0.1 to 0.6% w/v.

Adjuvanting immunogenic and vaccine compositions according to the invention may also be made with formulating them in the form of emulsions, in particular oil-in-water emulsions, e.g. an emulsion such as the SPT emulsion described p 147 in " Vaccine Design, The Subunit and Adjuvant Approach " edited by M. Powell, M. Newman, Plenum Press 1995, or the emulsion MF59 described p183 in the same book. In particular, the oil-in-water emulsion may be based on light liquid parafin oil (according to European Pharmacopoeia) ; isoprenoid oil such as squalane, squalene ; oil obtained by oligomerisation of alkenes, in particular of isobutylene or of decene ; acid or alcohol esters with linear alkyl groups, particularly vegetable oils, ethyl oleate, propylene glycol di(caprylate / caprate), glycerol tri(caprylate / caprate), propylene glycol dioleate; esters of branched fatty acids or alcohols, in particular esters of isostearic acid. The oil is used in combination with emulsifiers to form the emulsion. Emulsifiers are preferably non-ionic surfactants, in particular sorbitan esters, mannide esters, glycerol esters, polyglycerol esters, propylene glycol esters or esters of oleic acid, of isostearic acid, of ricinoleic acid, of hydroxystearic acid, possibly ethoxylated, blockcopolymers such as polyoxypropylene-polyoxyethylene, in particular the products called Pluronic(, namely Pluronic( L121.

From this disclosure and the knowledge in the art, the skilled artisan can select a suitable adjuvant, if desired, and the amount thereof to employ in an immunological, immunogenic or vaccine composition according to the invention, without undue experimentation.

The immunological or vaccine compositions according to the invention may be associated to at least one live attenuated, inactivated, or sub-unit vaccine, or recombinant vaccine (e.g. poxvirus as vector or DNA plasmid) expressing at least one immunogen, antigen or epitope of interest from another pathogen.

Compositions in forms for various administration routes are envisioned by the invention. And again, the effective dosage and route of administration are determined by known factors, such as age, weight. Dosages of each active agent e.g., of each *C. parvum* antigen or epitope of interest and/or of each antigen or epitope from each enteric pathogen can be as in herein cited documents or as otherwise mentioned herein and/or can range from one or a few to a few hundred or thousand micrograms, e.g., 1 µg to 1mg, for a subunit immunogenic, immunological or vaccine composition; and, 10⁴ to 10¹⁰ TCID₅₀ advantageously 10⁶ to 10⁸ TCID₅₀, before inactivation, for an inactivated immunogenic, immunological or vaccine composition.

Recombinants or vectors can be administered in a suitable amount to obtain *in vivo* expression corresponding to the dosages described herein and/or in herein cited documents. For instance, suitable ranges for viral suspensions can be determined empirically. The viral vector or recombinant for use in the composition of invention can be administered to the animal or infected or transfected into cells in an amount of about at least 10³ pfu; more preferably about 10⁴ pfu to about 10¹⁰ pfu, e.g., about 10⁵ pfu to about 10⁹ pfu, for instance about 10⁶ pfu to about 10⁸ pfu, with doses generally ranging from about 10⁶ to about 10¹⁰, preferably about 10¹⁰ pfu/dose, and advantageously about 10⁸ pfu per dose of about 1 ml to about 5 ml, advantageously about 2 ml. And, if more than one gene product is expressed by more than one recombinant, each recombinant can be administered in these amounts; or, each recombinant can be administered such that there is, in combination, a sum of recombinants comprising these amounts. In plasmid compositions employed in the invention, dosages can be as described' in documents cited herein or as described herein. Advantageously, the dosage should be a sufficient amount of plasmid to elicit a response analogous to compositions wherein the antigen(s) or epitope(s) of interest are directly present; or to have expression analogous to dosages in such compositions; or to have expression analogous to expression obtained *in vivo* by recombinant compositions. For instance, suitable quantities of each plasmid DNA in plasmid compositions can be 1 µg to 2 mg, preferably 50 µg to 1mg. Documents cited herein regarding DNA plasmid vectors may be consulted by the skilled artisan to ascertain other suitable dosages for DNA plasmid vector for use in compositions of the invention, without undue experimentation.

However, the dosage of the composition(s), concentration of components therein and timing of administering the composition(s), which elicit a suitable immunological response, can be determined by methods such as by antibody titrations of sera, e.g., by ELISA and/or seroneutralization and/or seroprotection assay analysis. Such determinations do not require undue experimentation from the knowledge of the skilled artisan, this disclosure and the documents cited herein. And, the time for sequential administrations can be likewise ascertained with methods ascertainable from this disclosure, and the knowledge in the art, without undue experimentation.

Preferably, the combined enteric immunological or vaccine composition comprises both *Cryptosporidium parvum* antigens as defined above.

Antigens or epitopes of enteric pathogens advantageously combined with *Cryptosporidium* antigen(s) or epitope(s) (advantageously P21 and/or Cp23 and/or Cp15/60 and/or CP41 such as P21 or Cp23 and Cp15/60, or epitope(s) thereof) may comprise one or more antigen or epitope of interest from *E. coli,* and/or rotavirus, and/or coronavirus, and/or *Clostridium spp.,* such as *Cl. perfringens*; for instance, at least one antigen or epitope of interest from *E. coli,* rotavirus, and coronavirus. Antigens from *E. coli.* include preferably one, preferably several (more than one), more preferably all, of the antigens called K99, F41, Y and 31A and/or epitopes therefrom. Preferred antigens are K99 and F41. A composition thus advantageously comprises one of K99 and F41, and preferably both. It is also preferred for a composition to comprise also Y and/or 31A, advantageously Y and 31A. For instance, these antigens may be incorporated as subunits or can be borne by *E. coli* bacteria. Preferably the compositions according to the invention comprise at least one antigen chosen from the group consisting of *E. coli* bearing K99 antigen, *E. coli.* bearing F41 antigen, *E. coli* bearing Y antigen, *E. coli* bearing 31A antigen, K99 antigen, F41 antigen, Y antigen, 31A antigen and any mixtures thereof.

As mentioned herein, *E. coli* may be used to produce *Cryptosporidium parvum* antigens or epitopes. The *Cryptosporidium parvum* antigens or epitopes can be expressed in an *E. coli* strain expressing at least one of the *E. coli* antigens so that simultaneous expression of *E. coli* and *Cryptosporidium parvum* antigens is performed. For *in vitro* expression, the cells may then be disrupted as usual and the *E. coli* and *Cryptosporidium parvum* antigens or epitopes recovered; advantageously, if there is internal or non-surface expression of the antigens or epitopes, the antigens or epitopes are expressed as fusion proteins or with tags, e.g. HIS tags. For *in vivo* expression, advantageously the nucleic acid molecules encoding the antigens or epitopes is linked to a signal sequence so that there is extracellular expression of the antigens or epitopes; and, advantageously, the *E. coli* is non-pathogenic, Thus, *E. coli* can, in certain embodiments, be the vector and the antigen or epitope of interest.

By way of information antigens from *Clostridium perfringens* are preferably type C and/or D toxoids, more preferably type C and D toxoids.

There is mentioned herein a combined enteric immunological, immunogenic or vaccine composition for bovine species, comprising at least one antigen or epitope from at least one C*ryptosporidium spp*., preferably, including *Cryptosporidium parvum*, advantageously P21 and/or Cp23 and/or Cp15/60 and/or CP41 such as P21 or Cp23 and Cp15/60 and/or an epitope of interest thereof, and at least one antigen or epitope from at least one additional bovine enteric pathogen such as *E. coli*, bovine rotavirus, bovine coronavirus and *Clostridium perfringens,* or combinations thereof, and preferably including at least one antigen or epitope from each of these pathogens or at least one antigen or epitope from *E. coli,* rotavirus, and coronavirus. With respect to an epitope of interest of a desired antigen and how to determine what portion of an antigen is an epitope of interest, reference is made to U.S. Patent Nos. 5,990,091, 6,090,393 and 6,156, 567. From the disclosure herein and the knowledge in the art, such as in herein cited documents, there is no undue experimentation needed to ascertain an epitope of interest, or to formulate a composition within the invention comprising antigen(s) and/or epitope(s) and/or vector(s) expressing antigen(s) and/or epitope(s).

According to a preferred embodiment, the invention provides a bovine enteric immunological or vaccine composition comprising *E. coli* antigens as discussed herein such as antigens K99, F41, Y and 31A, as well as inactivated bovine coronavirus, inactivated bovine rotavirus. This composition can further include *Clostridium perfringens* type C and D toxoids. Preferably the *E. coli* valency comprises either inactivated *E. coli* bearing K99 antigen, inactivated *E. coli*. bearing F41 antigen, inactivated *E. coli* bearing Y antigen and inactivated *E. coli* bearing 31 A antigen, or, K99 antigen, F41 antigen, Y antigen and 31A antigen.

Another aspect of the present invention is an immunological or vaccine composition against *Cryptosporidium parvum*, which comprises Cp23 or P21 and Cp15/60 antigens or epitopes thereof, and a pharmaceutically acceptable vehicle.

According to an advantageous embodiment, these antigens are incorporated in the composition as proteins or sub-unit antigens. They can be produced by chemical synthesis or by expression *in vitro.* For the convenience of production by expression in a suitable host, and their recovery, these antigens are preferably under the form of fusion protein (e.g., with HIS tag). In other words, the antigen can comprise the antigen *per se* and foreign amino acids.

According to another embodiment, these antigens are not incorporated as subunits in the composition, but the composition comprises either a recombinant vector expressing Cp23 or P21 and Cp15/60 or an epitope thereof or a recombinant vector expressing Cp23 or P21 or an epitope thereof and a recombinant vector expressing Cp15/60 or an epitope thereof, wherein these vectors express the antigen(s) or epitope(s) *in vivo* when administered to the animal. The composition can contain an antigen or epitope and a vector expressing the other antigen or epitope.

There is mentioned herein methods of vaccination wherein one administers to a target animal a combined enteric immunological or vaccine composition or an immunological or vaccine composition against *Cryptosporidium parvum* according to the invention. There is discussed herein a method of immunization of a new-born calf against enteric disease, comprising administering an immunological or vaccine composition comprising Cp23 or P21 and Cp15/60 *Cryptosporidium parvum* antigens or epitopes thereof and a pharmaceutically acceptable vehicle, to the pregnant cow or pregnant heifer before delivering, so that the newborn calf has maternal antibodies against *Cryptosporidium parvum.* Preferably, the method comprises the feeding of the newborn calf with colostrum and/or milk coming from a cow, e.g. the mother, which has been so vaccinated. For vaccination or immunization against enteric disease, one may not only use a combined vaccine, immunogenic or immunological composition, containing the various valencies, but also separate vaccine, immunogenic or immunological compositions which can be administered separately, e.g., sequentially, or which can be mixed before use.

Antigens and epitopes of interest useful in inventive compositions and methods may be produced using any method available to the one skilled in the art and for instance using the methods in US-A-5,591,434 and WO-A-9807320. Further, one can obtain antigens of other enteric pathogens from commercially available sources, such as TRIVACTON®6; for instance, Cp23 and/or P21 and/or Cp15/60 or an epitope thereof, e.g., P21 or Cp23 and Cp15/60 or an epitope thereof, or a vector expressing these antigen(s) or epitope(s) can be added to TRIVACTON®6, in herein specificed amounts. *Clostridium perfringens* toxoids C and D may be added to TRIVACTON®6. Also, the inactivated *E. coli* bearing pili may be replaced in TRIVACTON®6 by the isolated pili. Such a vaccine, immunogenic or immunological composition (with inactivated *E. coli* or isolated pili) to which *C. parvum* antigen(s) or epitope(s) and/or *Clostridium perfringens* antigen(s) or epitope(s) is/are added and methods of making and using such a composition and kits therefor are also mentioned herein.

Furthermore, as to the *E. coli* valency and/or antigen(s) and/or epitope(s) useful in the practice of the invention, reference is made to EP-A-80,412, EP-A-60,129, GB-A-2,094,314, and U.S. Patents Nos. 4,298,597, 5,804,198, 4,788,056, 3,975,517, 4,237,115, 3,907.987, 4,338,298, 4,443,547, 4,343,792, 4,788,056, and 4,311,797. As to rotavirus antigen(s) and/or epitope(s), reference is made to P.S. Paul and Y.S. Lyoo, Vet Microb 37:299-317 (1993) and U.S. Patents Nos. 3,914,408 and 5,620,896. With respect to coronavirus antigen(s) and/or epitope(s), reference is made to WO-A-98 40097, WO-A-96 41874, and U.S. Patents Nos. 3,914,408 and 3,919,413. For *Clostridium, e.g., Cl. perfringens,* antigen(s) and/or epitope(s), reference is made to WO-A-94 22476, EP-A-734,731, WO-A-98 27964, GB-A-2,050,830, GB-A-1,128,325, D. Calmels and Ph. Desmettre, IV Symposium of the Commission for the study of animal diseases caused by anaerobes, Paris, Nov. 16-18, 1982, U.S. Patents Nos. 5,178,860, 4,981,684, and 4,292,307; and, to IMOTOXAN® (MERIAL, Lyon, France) (containing types B, C, D, Cl. *perfringens*, toxoids from *Cl. septicum, Cl. novyi, Cl. tetani* and culture of *Cl. chauvoei*). And, in addition to TRIVACTON®6, one may use other commercial combined vaccines to which *C. parvum* valency can be added, for instance, SCOURGUARD 3 (K)/C® (SmithKline Beecham) containing inactivated bovine rotavirus and coronavirus, K99 *E. coli* bacterin and *Cl. perfringens* type C toxoid.

A preferred method to obtain antigens or epitopes of interest is to clone the DNA sequence encoding the antigen or epitope of interest into a fusion or non-fusion plasmid and to have its expression in *E. coli*. Fusion plasmids (e.g., that express the antigen(s) or epitope(s) with a tag such as a His tag) are preferred as they allow one to recover easily the produced antigen. Suitable plasmids are described in the examples. Production of antigens by chemical synthesis is mentioned herein.

There is mentioned herein methods for using herein discussed antigens or epitopes or vectors expressing such antigens or epitopes for the preparation of a vaccine, immunological, or immunogenic composition, e.g., against *C. parvum* or against enteric disease; for instance, by admixing the antigens, epitopes or vectors with a suitable or acceptable carrier or diluent and optionally also with an adjuvant. The compositions may be lyophilized for reconstitution. The invention further comprehends a kit for the preparation of an inventive composition. The kit can comprise the antigen(s), epitope(s) and/or vector(s), carrier and/or diluent and optionally adjuvant; the ingredients can be in separate containers. The containers containing the ingredients can be within one or more than one package; and, the kit can include instructions for admixture of ingredients and/or administration of the vaccine, immunogenic or immunological composition composition.

There is also described herein the production of hyperimmune colostrum and/or milk; for instance, by hyperimmunization of the pregnant female mammal (such as a cow) by at least 1, advantageously at least 2, and more advantageously at least 3, administrations of inventive composition(s) (*e.g.*, *C. parvum* composition or combined enteric composition according to the invention). Optionally, but advantageously, the colostrum and/or milk so produced can then be treated to concentrate the immunoglobulins and to eliminate components of the colostrum or milk that do not contribute to the desired immunological, immunogenic and/or vaccine response or to the nutritional value of the colostrum or milk. That treatment can advantageously comprise coagulation of the colostrum or milk, e.g., with rennet, and the liquid phase containing the immunoglobins recovered. There is mentioned herein hyperimmune colostrum or milk or mixture thereof and/or compositions comprising the hyperimmune colostrum or milk or mixture thereof. Further, there is described herein the use of the hyperimmune colostrum or milk or mixture thereof or composition comprising the same to prevent or treat *C. parvum* and/or enteric infection in a young animal, such as a new-born; for instance, a calf.

Accordingly, the invention shall be further described by way of the following Examples, provided for illustration and not to be considered a limitation of the invention.

### EXAMPLES

| **List of sequences :** | |
|---|---|
| SEQ ID NO: 1 | oligonucleotide JCA295 |
| SEQ ID NO: 2 | oligonucleotide JCA296 |
| SEQ ID NO: 3 | oligonucleotide JCA297 |
| SEQ ID NO: 4 | oligonucleotide JCA298 |
| SEQ ID NO: 5 | oligonucleotide JCA299 |
| SEQ ID NO: 6 | oligonucleotide JCA300 |
| SEQ ID NO: 7 | oligonucleotide JCA301 |
| SEQ ID NO: 8 | oligonucleotide JCA302 |
| SEQ ID NO: 9 | oligonucleotide JCA303 |
| SEQ ID NO: 10 | oligonucleotide JCA304 |

All plasmid constructs have been done using standard molecular biology techniques (cloning, restriction digestion, polymerase chain reaction (PCR)) as described in Sambrook J. et al. (Molecular Cloning: A Laboratory Manual. 2nd Edition. Cold Spring Harbor Laboratory. Cold Spring Harbor. New York. 1989). All DNA restriction fragments generated and used for the present invention, as well as PCR fragments, have been isolated and purified using the "Geneclean®" kit (BIO101 Inc. La Jolla, CA).

### Example 1: Cloning of the C. parvum P21 and Cp15/60 genes

Oocysts of *Cryptosporidium parvum* are isolated from an infected calf and are purified from bovine fecal samples as described by Sagodira S. et al. (Vaccine. 1999. 17. 2346-2355). Purified oocysts are then stored in distilled water at +4°C. For use as a template for PCR reactions, genomic DNA is released from the purified oocysts as described by Iochmann S. et al. (Microbial Pathogenesis 1999. 26. 307-315).

An alternative source for *C. parvum* DNA is constituted by the EcoRI genomic libraries for the *Cryptosporidium parvum* Iowa (A), Iowa (I), KSU-1 and KSU-2 isolates available from the American Tissue Culture Collection (ATCC numbers 87667, 87668, 87439 and S7664 respectively). The specific P21and Cp15/60 genes are isolated as follows :
The sequence encoding the P21 protein is amplified by a polymerase chain reaction (PCR) using *C. parvum* DNA and the following primers:
   oligonucleotide JCA295 (35 mer) SEQ ID NO: 1
      5' TTT TTT CCA TGG GGC TCG AGT TTT CGC TTG TGT TG 3'
   and oligonucleotide JCA296 (33 mer) SEQ ID NO: 2
      5' TTT TTT GAA TTC TTA GGC ATC AGC TGG CTT GTC 3'

This PCR generates a fragment of about 585 bp PCR fragment. This PCR fragment is then digested with NcoI and EcoRI restriction enzymes to isolate, after agarose gel electrophoresis and recovery with the GeneClean kit (BIO101 Inc.), a 575 bp NcoI-EcoRl restriction fragment (= fragment A). The sequence of this fragment encodes a protein homologous to the sequence described as SEQ ID NO: 12 in patent application WO 98/07320 (PCT/US97/14834).

A second PCR is run to amplify the sequence encoding the Cp15/60 protein and to add convenient restriction sites in 5' and 3' for further cloning. The PCR is done using *C. parvum* DNA and the following primers:
oligonucleotide JCA297 (35 mcr) SEQ ID NO: 3
   5' TTT TTT CTC GAG ATG GGT AAC TTG AAA TCC TGT TG 3'
and oligonucleotide JCA298 (42 mer) SEQ ID NO: 4
5' TTT TTT GAA TTC TTA GTT AAA GTT TGG TTT GAA TTT GTT TGC 3'

This PCR generates a fragment of about 465 bp. This fragment is purified and then digested with XhoI and EcoRI in order to get, after agarose gel electrophoresis and recovery with the GeneClean kit (BIO101 Inc.), the 453 bp XhoI-EcoRI fragment (= fragment B). The amplified sequence is homologous to be similar to the sequence defined from nucleotide #31 to #528 of SEQ ID NO: 1 in US Patent # 5,591,434 and to the sequences deposited in GenBank under Accession Numbers U22892 and AAC47447.

### Example 2: Construction of plasmid pJCA155

### (GST-P21 fusion protein in vector pBAD/HisA)

The sequences required to express the GST-P21 fusion protein are amplified by PCR in order to generate 2 fragments that can be cloned easily into the pBAD/HisA expression plasmid vector (Cat # V430-01 InVitrogen Corp., Carlsbad, CA 92008, USA). The first PCR is done using the pGEX-2TK plasmid (Cat # 27-4587-01 Amersham-Pharmacia Biotech) and the following primers:
oligonucleotide JCA299 (35 mer) SEQ ID NO: 5
   5' TTT TTT CCA TGG GGT CCC CTA TAC TAG GTT ATT GG 3'
and oligonucleotide JCA300 (45 mer) SEQ ID NO: 6
   5' TTT TTT CTC GAG CCT GCA GCC CGG GGA TCC AAC AGA TGC ACG ACG 3'

This PCR generates a fragment of about 720 bp encoding the GST moiety with the addition of a NcoI restriction site at the 5' end for cloning purposes into pBAD/HisA; this modification adds a Glycine codon to the GST-P21 fusion protein). This PCR fragment is then digested with NcoI and XhoI in order to get, after agarose gel electrophoresis and recovery with the GeneClean kit (BIO101 Inc.), the 710 bp NcoI-XhoI fragment (= fragment C).

The second PCR is done using *C. parvum* DNA and the following primers:
oligonucleotide JCA301 (33 mer) SEQ ID NO: 7
   5' TTT TTT CTC GAG TTT TCG CTT GTG TTG TAC AGC 3'
and oligonucleotide JCA296 (33 mer) SEQ ID NO: 2

This PCR generates a fragment of about 580 bp encoding the P21 moiety with the addition of XhoI and EcoRI restriction sites at the 5' and 3' ends respectively. This PCR fragment is then digested with XhoI and EcoRI in order to get, after agarose gel electrophoresis and recovery with the GeneClean kit (BIO101 Inc.), the 572 bp XhoI-EcoRI fragment (= fragment D).

The pBAD/HisA plasmid (Cat # V430-01, InVitrogen Corp.) is digested with NcoI and EcoRI. The digested fragments are separated by agarose gel electrophoresis in order to recover (GeneClean kit, BIO101 Inc.) the # 3960 bp NcoI-EcoRI restriction fragment (= fragment E).

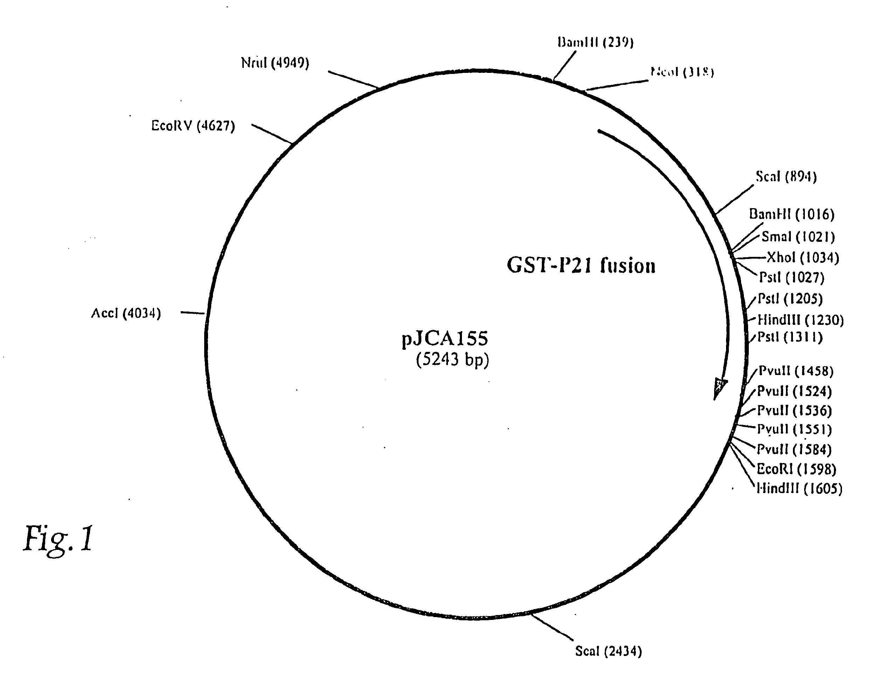

Fragments C, D and E are then ligated together to generate plasmid pJCA155. This plasmid has a total size of 5243 bp (Figure 1) and encodes a 425 amino acids GST-P21 fusion protein.

### Example 3: Construction of plasmid pJCA156

### (His6-P21 fusion protein in vector pBAD/HisA)

The pBAD/HisA vector (Cat # V430-01, InVitrogen) is digested with NcoI and EcoRI and the # 3960 bp NcoI-EcoRI restriction fragment (= fragment E) is recovered and isolated as described in Example 2.

A PCR is done to amplify the sequence encoding the His6-P21 fusion and to add the NcoI and EcoRI restriction sites respectively in 5' and 3' in order to subclone this PCR fragment into the pBAD/HisA plasmid vector.

The PCR is done using *C. parvum* DNA and the following primers:
oligonucleotide JCA302 (65 mer) SEQ ID NO: 8
and oligonucleotide JCA296 (33 mer) SEQ ID NO: 2

This PCR generates a fragment of about 610 bp. This fragment is purified, and then digested with NcoI and EcoRI in order to isolate, after agarose gel electrophoresis and recovery with the GeneClean kit (BIO101 Inc.), the 600 bp NcoI-EcoRI fragment (= fragment F).

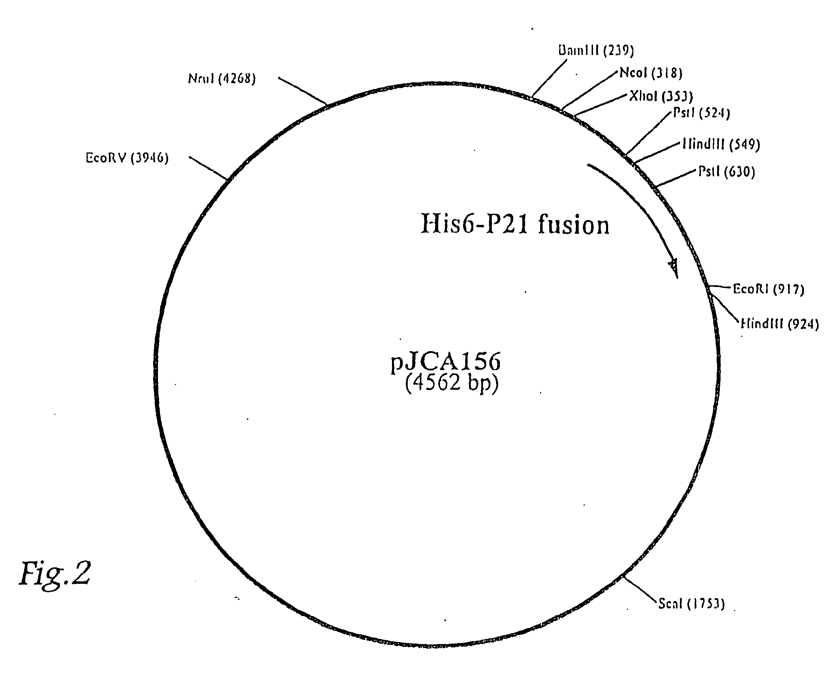

Fragments E and F are ligated together to generate plasmid pJCA156. This plasmid has a total size of 4562 bp (Figure 2) and encodes a 199 amino acids His-6/P21 fusion protein. **Example 4: Construction of plasmid pJCA157**

### (P21 protein alone in pBAD/HisA vector)

The pBAD/HisA vector (Cat # V430-01, In Vitrogen Corp.) is digested with NcoI and EcoRI and the # 3960 bp NcoI-EcoRI restriction fragment (= fragment E) is recovered and isolated as described in Example 3.

A PCR is done to amplify the sequence encoding the P21 protein and to add the NcoI and EcoRI restriction sites respectively in 5' and 3' in order to subclone this PCR fragment into the pBAD/HisA plasmid vector. The PCR is done using *C. parvum* DNA and the following primers:
oligonucleotide JCA295 (35 mer) SEQ ID NO: 1
and oligonucleotide JCA296 (33 mer) SEQ ID NO: 2
to get, as described in Example 1, a 575 bp NcoI-EcoRI fragment (fragment A).

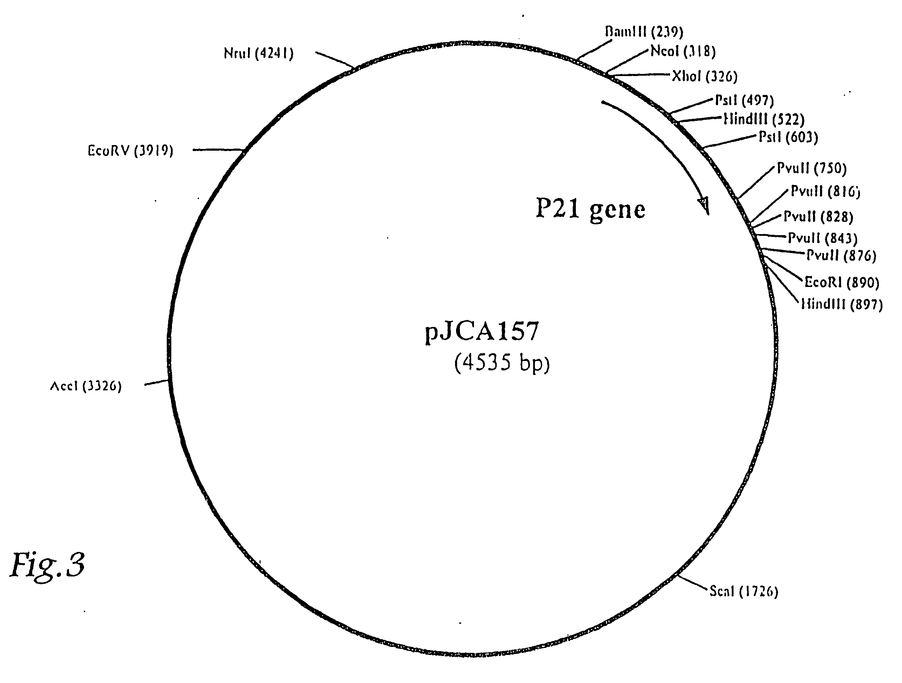

Fragments E and A are ligated together in order to generate plasmid pJCA157. This plasmid has a total size of 4535 bp (Figure 3) and encodes 189 amino acids including the P21 protein.

### Example 5: Construction of plasmid pJCA158

### (GST-Cp15/60 fusion protein in pBAD/HisA vector)

A PCR is done to amplify the sequence encoding the GST protein and to add convenient restriction sites in 5' and 3' in order to subclone the PCR fragment into the final pBAD/HisA plasmid vector. The PCR uses the DNA of plasmid pGEX-2TK (Cat # 27-4587-01, Amersham-Pharmacia Biotech) as a template and the following primers:
oligonucleotide JCA299 (35 mer) SEQ ID NO: 5
and oligonucleotide JCA300 (45 mer) SEQ ID NO: 6
to get, as described in example 2, a 710 bp NcoI-XhoI fragment (= fragment C).

The pBAD/HisA vector (Cat # V430-01, InVitrogen) is digested with NcoI and EcoRI and the # 3960 bp NcoI-EcoRI restriction fragment (= fragment E) is recovered and isolated as described in Example 2.

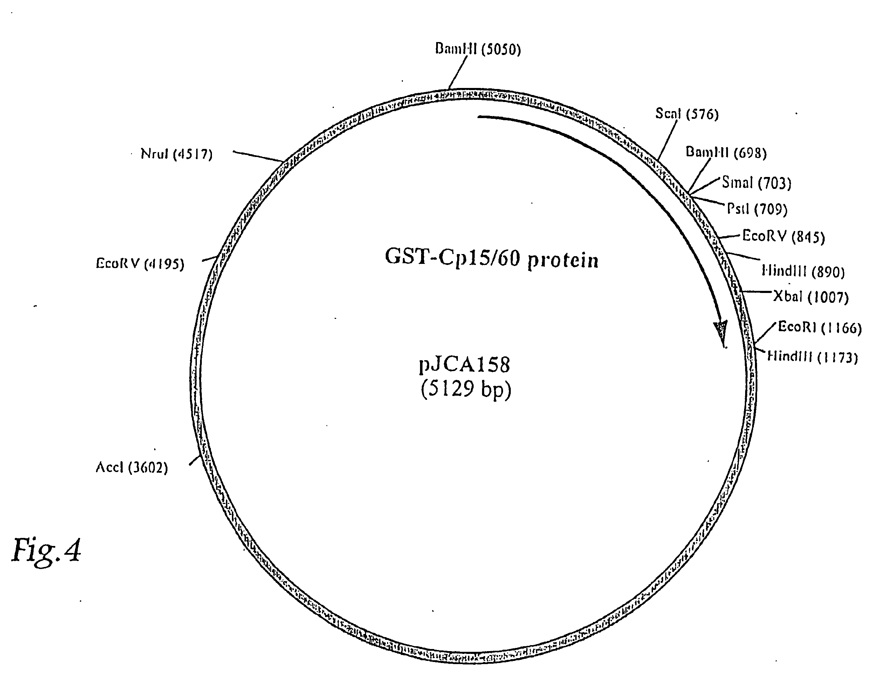

Fragments C, E and B (Example 1) are ligated together in order to generate plasmid pJCA158. This plasmid has a total size of 5132 bp (Figure 4) and expresses a 388 amino acids GST-Cp15/60 fusion protein.

### Example 6: Construction of plasmid pJCA159

### (His6-Cp15/60 fusion protein in pBAD/HisA vector)

The pBAD/HisA vector (Cat # V430-01, InVitrogen Corp.) is digested with NcoI and EcoRI and the # 3960 bp NcoI-EcoRI restriction fragment (= fragment E) is recovered and isolated as described in Example 2.

A PCR is run to amplify the sequence encoding the His6-Cp15/60 fusion and to add convenient restriction sites in 5' and 3' in order to subclone this PCR fragment into the pBAD/HisA plasmid vector. The PCR is done using either *C. parvum* DNA and the following primers:
oligonucleotide JCA303 (64 mer) SEQ ID NO: 9
and oligonucleotide JCA298 (42 mer) SEQ ID NO: 4

This PCR generates a fragment of about 495 bp. This fragment is purified and then digested with NcoI and EcoRI in order to get, after agarose gel electrophoresis and recovery with the GeneClean kit (BIO101 Inc.), the 483 bp NcoI-EcoRI fragment (= fragment G).

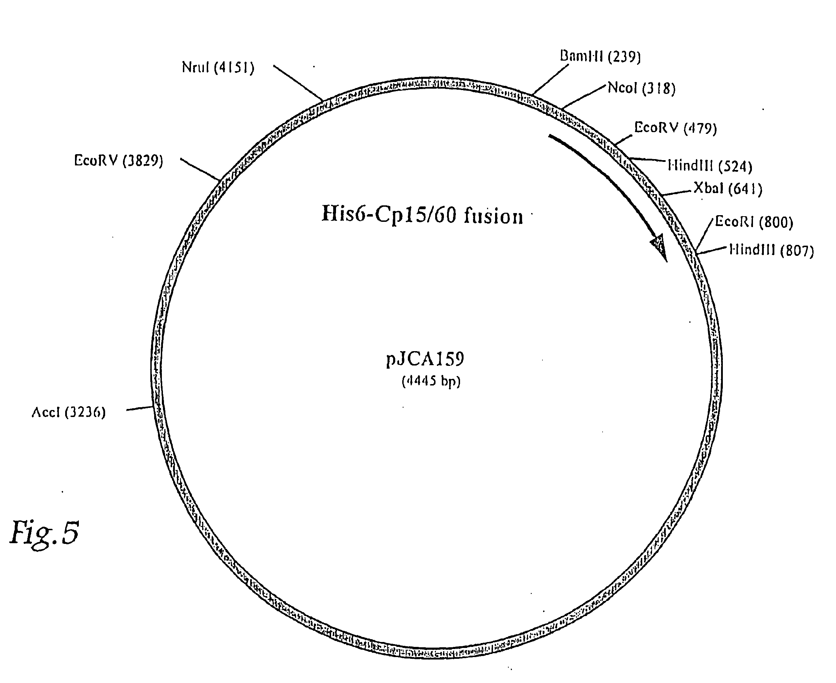

Fragments E and G are ligated together in order to generate plasmid pJCA159. This plasmid has a total size of 4445 bp (Figure 5) and expresses a 159 amino acids His-6/Cp15/60 fusion protein.

### Example 7: Construction of plasmid pJCA160

### (Cp15/60 protein alone in pBAD/HisA vector)

The pBAD/HisA vector (Cat # V430-01, In Vitrogen Corp.) is digested with NcoI and EcoRI and the # 3960 bp NcoI-EcoRI restriction fragment (= fragment E) is recovered and isolated as described in Example 2.

A PCR is run to amplify the sequence encoding the Cp15/60 protein and to add convenient restriction sites in 5' and 3' in order to subclone this PCR fragment into the pBAD/HisA plasmid vector.

The PCR is done using *C. parvum* DNA and the following primers:
oligonucleotide JCA304 (31 mer) SEQ ID NO: 10
   5' TTT TTT CCA TGG GTA ACT TGA AAT CCT GTT G 3'
and oligonucleotide JCA298 (42 mer) SEQ ID NO: 4

This PCR generates a fragment of about 460 bp. This fragment is purified and then digested with NcoI and EcoRI in order to get, after agarose gel electrophoresis and recovery with the GeneClean kit (BIO101 Inc.), the 450 bp NcoI-EcoRI fragment (= fragment H).

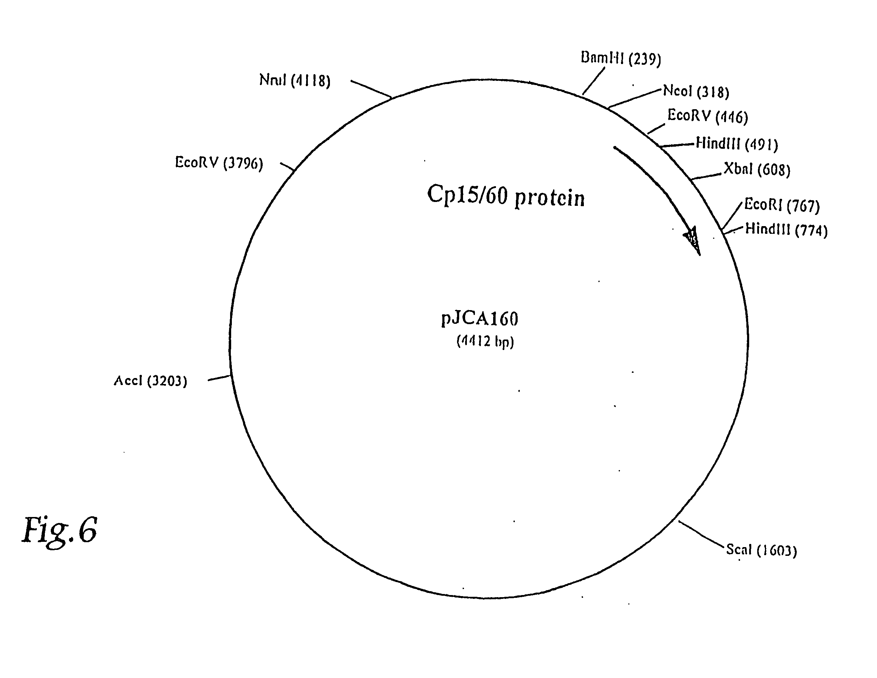

Fragments E and H are ligated together in order to generate plasmid pJCA160. This plasmid has a total size of 4412 bp (Figure 6) and expresses a 148 amino acids Cp15/60 protein.

### Example 8: Culture of E. coli recombinant clones

### and induction of recombinant proteins

Plasmid DNA (Examples 2 to 7) is transformed into *Escherichia coli* DH5α (or any other suitable *E. coli* K12 strain well known to those skilled in the art, such as *E. coli* TOP10 (Cat # C4040-03 InVitrogen Corp.)) and grown on Luria-Bertani (LB) medium agar plates with 50µg/ml of ampicillin. One colony is picked for each plasmid transformed *E. coli* population and placed in 10 ml of LB medium with ampicillin (or other appropriate antibiotic) for overnight growth. One ml from the overnight culture is added to one liter of LB medium and grown at +30°C until OD_{600 nm} reaches approximately 3.0.

Protein production is induced with different final concentrations of DL-arabinose (Cat# A9524, Sigma, St Louis, MO) (range of 0.002% to 0.2% for determining the concentration for optimal yield) added to the culture and incubated at +30°C for 4-6 hours.

### Example 9: Extraction and purification of the recombinant fusion proteins

At the end of the induction (Example 8), cells are harvested by centrifugation (3000 g, 10 minutes, +4°C) and resuspended in lysis buffer (50 mM Tris pH 8.0, 1 mM EDTA, 1 µM PMSF, 1 mg/ml lysozyme) and sonicated 25 times for 30 seconds bursts with 1 minute pauses between bursts. Triton X-100 is added to a final concentration of 0.1%. Debris are removed by centrifugation.

If necessary, alternative techniques (known to those of skill in the art) may be used for the lysis of bacterial cells.

### 9.1. GST-fusion recombinant proteins:

Recombinant GST-fusion proteins (produced by *E. coli* transformed with plasmids pJCA155 or pJCA158) were affinity purified from the bacterial lysates, prepared as described in Example 8, using a glutathione-agarose (Cat# G4510, Sigma) or glutathione-Sepharose 4B (Cat# 17-0756-01, Amersham-Pharmacia Biotech). Bacterial lysates and the glutathione-agarose were incubated for 4 hours at +4°C. GST-fusion proteins were then eluted from the agarose in a batch format with 10 mM reduced form glutathione (Cat# G4705, Sigma) under mild conditions (K. Johnson and D. Smith Gene. 1988. 67. 31-40). (Reference : Anonymous. GST gene fusion system : technical manual. 3rd edition. Arlington Heights, IL: Amersham-Pharmacia Biotech, 1997). Anyone skilled in the art can achieve scaling up of this process for purifying large quantities of GST-fusion proteins, from this disclosure and the knowledge in the art, without undue experimentation.

### 9.2. His6-fusion recombinant proteins:

Recombinant His6-fusion proteins have all been prepared and purified using the ProBond™ Nickel-Chelating resin (Cat# R801-15, InVitrogen Corp.) following the manufacturer's instructions.

Preparation of native *E. coli* cell lysate (soluble recombinant protein) : the bacterial cells from a 1 liter culture of *E. coli* (transformed with plasmids pJCA156 or pJCA159) are harvested by centrifugation (3000 g for 5 minutes). The pellet is resuspended in 200 ml of Native Binding Buffer (20 mM phosphate, 500 mM NaCl, pH 7.8). The resuspended pellet is then incubated with egg lysozyme at a final concentration of 100 µg/ml, for 15 minutes on ice. This mixture is then sonicated with 2-3 10-second bursts at medium intensity while holding the suspension on ice. The mixture is then submitted to a series of freezing/thawing cycles for completing the lysis and the insoluble debris are finally removed by centrifugation at 3000 g for 15 minutes. The lysate is cleared by passage through a 0.8 µm filter and stored on ice or at -20°C until purification.

The soluble recombinant His6-fusion protein present in the clear lysate is batch bound to a 50 ml pre-equilibrated ProBond™ resin column (Cat # R640-50 and R801-15, InVitrogen Corp.) with two 100 ml lysate aliquots. The column is gently rocked for 10 minutes to keep the resin resuspended and allow the polyhistidine-tagged protein to fully bind. The resin is settled by gravity or low speed centrifugation (800 g) and the supernatant is carefully aspirated. An identical cycle is repeated with the second aliquot.

### Column washing and elution :

4 successive steps are done according to the manufacturer's instructions (Anonymous. Xpress™ System Protein Purification - A Manual of Methods for Purification of Polyhistidine - Containing Recombinant Proteins. InVitrogen Corp. Editor. Version D. 1998):
1. The column is washed with 100 ml of Native Binding Buffer pH 7.8, by resuspending the resin, rocking for 2 minutes and then separating the resin from the supernatant by gravity or centrifugation. This procedure is repeated 2 more times (total of 3 washes)
2. The column is washed with 100 ml of Native Wash Buffer pH 6.0 by resuspending the resin, rocking for 2 minutes and then separating the resin from the supernatant by gravity or centrifugation. This procedure is repeated at least 3 more times until OD₂₈₀ is less than 0.01.
3. The column is washed with 100 ml of Native Wash Buffer pH 5.5 by resuspending the resin, rocking for 2 minutes and then separating the resin from the supernatant by gravity or centrifugation. This procedure is repeated once (total of 2 washes).
4. The column is then clamped in vertical position and the cap is snapped off on the lower end. The recombinant protein is eluted with 150 ml of the Native pH Elution Buffer. 10 ml fractions are collected. Elution is monitored by taking OD₂₈₀ readings of the fractions.
If needed, the eluted recombinant protein can be concentrated either by dialysis, or by precipitation with ammonium sulfate.

Final concentration of the recombinant protein batch is measured by OD₂₈₀ readings.

Anyone skilled in the art can achieve scaling up of this process for purifying large quantities of His6-fusion proteins, from this disclosure and the knowledge in the art, without undue experimentation.

### Example 10: Extraction and purification of the C. parvum

### P21 and Cp15 recombinant non-fusion proteins

The bacterial cells of *E. coli* (transformed with plasmids pJCA157 or pJCA160) are cultured in 4 liters of the M9 minimum medium (supplemented with the appropriate amino acids) (Sambrook J. et al. (Molecular Cloning: A Laboratory Manual. 2nd Edition. Cold Spring Harbor Laboratory. Cold Spring Harbor. New York. 1989) at 30°C until OD_{600 nm} reaches approximately 3.0 and arc induced as described in Example 8. The bacterial cells are then disrupted by passing through a high pressure RANNIE homogeneizer Mini-Lab type 8.30 H with a maximum flow of 10 liters per hour and working pressure between 0 and 1000 bars. The lysate is cleared by filtration through a CUNO filter Zeta plus, LP type, and then concentrated 50 times on an ultrafilter PALL Filtron (reference OS010G01) UF 10 kDa. The protein suspension concentrate is loaded on a size-exclusion chromatography column with High Resolution Sephacryl S-100 gel under a volume corresponding to 2-3% of the column volume. Elution is done with a PBS buffer. The collected fractions corresponding to the expected molecular weight for the subunit vaccine proteins are concentrated 10 times on a hollow fibers cartridge A/G Technology type Midgee cartridge model UFP-10-B-MB01 (or model UFP-10-C-MB01 or model UFP-10-E-MB01). The concentrated samples are then stored at -70°C until use. The specific *C. parvum* recombinant proteins can be then mixed in the appropriate proportions to the final associated vaccine (see Example 11).

### Example 11: Formulation of vaccines; vaccination of pregnant cows;

### passive immunization and challenge experiment in newborn calves

Product (adjuvanted or not) is administered intramuscular (IM), subcutaneous (SQ) or intradermal (ID) to elicit serum antibody responses against *C. parvum.* When administered twice to pregnant animals it elicits a serum antibody response that will be passively transferred to the newborn via colostrum and milk. Vaccination protocol for pregnant animals can comprise 2 doses given between when pregnancy is diagnosed and calving, such as about 1 month before calving and about 3 to 5 days before calving; or, 2 months prior to calving (which coincides with dry-off in dairy cows) and a boost prior to calving (e.g., anywhere from 3 weeks to 1 week prior to calving), depending on management practices (however, these schedules favor maximum efficacy). Current management practices favor that are products administered in the last trimester. Volume of the product can be from 1 ml to 5 ml, such as 2 ml. Combination vaccines can have a lyophylized and a liquid portion that can be mixed prior to injection. To afford maximum protection under field conditions the *Cryptosporidium* antigen can be added as a component of an *E. coli*/Rota/Corona combination vaccine.

The following studies are conducted:

### Study A: C. parvum enhances the pathogenicity of enteric virus and/or bacteria

Experimental challenge utilizing 3 newborn calves per group as follow:
1. Coronavirus only
2. Coronavirus plus *C. parvum*
3. *E. coli* F41 only
4. *E. coli* F41 plus *C. parvum*
5. *C. parvum* only
6. Unchallenged controls

Calves are challenged within 24 hours of being born, by the oral route. The amount of challenge material used is that which is necessary to produce clinical signs (depression, diarrhea, dehydration) and may depend on the type of animal (gnotobiotic artificially raised or conventional calve nursing its dam). Common clinical signs (temperature, demeanor, hydration, diarrhea scores, etc.) are collected. Additional serological and shedding information is collected.

### Outcome

Coronavirus or *E. coli* F41 monovalent experimental challenges do not produce clinical signs of enteric disease in newborn calves. Dual challenge with coronavirus or *E. coli* F41 with *C. parvum,* at a *C. parvum* dose that normally does not cause clinical disease, will produce significant clinical signs of enteric disease.
**Study B: A combo vaccine (*E. coli* K99/F41, rota and coronavirus) containing *C. parvum* provides enhanced protection against enteric disease cause by concurrent infection of multiple enteric virus and/or bacteria in newborn calves.**

Treatment groups are 30 pregnant cows vaccinated with:
1. Combo (rota and coronavirus, *E. coli* K99 and F41), 8 animals;
2. Combo plus *Crypto,* 8 animals;
3. Unvaccinated controls, 14 animals.

### Experimental challenge as follow:

1. Multiple challenge (coronavirus and F41 plus *C. parvum* at subclinical level);
2. Sentinel animals
3. unchallenged.

Calves receive colostrum (manually fed or allowing the calve to nurse from the dam) and those that are challenged are challenged within 24 hours of being born, by the oral route. The amount of challenge material is an amount necessary to produce clinical signs (e.g., as determined in Study A, and as mentioned under Study A, can vary depending upon the type of animal used (e.g., gnotobiotic artificially raised or conventional calves nursing their dams). Common clinical signs (temperature, demeanor, diarrhea scores) are collected. Additional serological and shedding information is collected.

### Design:

6 calves born from vaccinated (combo and combo plus *Crypto*) or control cows are challenged with a challenge containing 3 components (coronavirus and F41 plus *C. parvum*), and 3 calves (from unvaccinated control cows) remain as sentinels.

### Outcome

Use of a combo vaccine containing *C. parvum* produces a better protection than a combo vaccine alone under a multiple challenge situation (coronavirus and *E. coli* F41 with *C. parvum* at a subclinical dose).

### Example 12 : Effect of dual infection with C. parvum and bovine rotavirus in an experimental challenge model in newborn calves

This study is designed to compare the severity of clinical signs and fecal excretion in calves after monovalent challenge with *C. parvum* or bovine rotavirus and after a dual challenge with bovine rotavirus plus *C. parvum*.

Four groups of six calves are used in order to yield sufficient data to be able to detect differences in incidence of clinical signs between groups.

Cows are individually housed in pens or paddocks. Newborn calves are separated from their dams as soon as possible after birth, inspected to eliminate feces or dirt on the calf and their ombilical cord dipped in approximate 7% iodine solution. They are then immediately transferred to containment accomodations and housed individually in metabolic crates. Calves are challenged within 6 hours after birth.

Calves are fed 1 to 2 quarts per feeding or at 10% body weight, twice daily for the entire trial using a commercial calf milk replacer with 30% colostrum substitute. Special care will be given to avoid the administration of milk within 2 hours pre or post challenge.

The route of natural infection is oral ; therefore, all the challenges will be administered orally using an esophageal tube.

Group A : non-challenged control calves.

Group B : 1-3x10⁵ *C. parvum* oocysts (strain Beltsville), diluted in 60 ml of commercial antibiotics free soy milk.

Group C : Coinoculation of 1-3x10⁵ *C. parvum* oocysts (strain Beltsville), diluted in 60 ml of commercial antibiotics free soy milk, and of 10 ml bovine rotavirus inoculum (strain IND BRV G6P5) diluted in 40 ml PBS.

Group D : 10 ml fecal filtrate from bovine rotavirus infected calves (strain IND BRV G6P5) diluted in 40 ml PBS.

Fecal samples are collected from the collection pan once a day after thoroughly mixing to ensure a representative sample is obtained.

Oocysts are separated from calves feces by centrifugation on sucrose cushions and counted using a cell counting chamber (hemocytometer) under a microscope. For rotavirus shedding, the feces are diluted in buffer and the rotavirus antigen is quantified using an ELISA kit from Le Centre d'Economie Rurale (CER) 1 rue du Carmel, B6900 Marloie, Belgium.

Calves are observed for clinical signs prior to challenge and then twice daily for 10 days post-challenge. Observations include rectal temperature, general condition, anorexia, diarrhea, dehydration and death.

Depression, diarrhea, and dehydration are categorized as follows :
General condition :

| | |
|---|---|
| Good | The calf is bright, alert and responsive |
| Apathic | The calf is quiet, alert and responsive |
| Depression | The calf is lying aside, reluctant to rise, and slow to respond |
| Prostration | The calf is curled up or prostrate and not responsive |

Dehydration :

| | |
|---|---|
| None | No dehydration |
| Moderate | Persistent skin fold, dry mouth and depressed eyeballs |
| Shock | State of shock |

Diarrhea:

| | |
|---|---|
| None | Normal feces |
| Loose | Pasty or mucous feces |
| Liquid | Liquid feces |

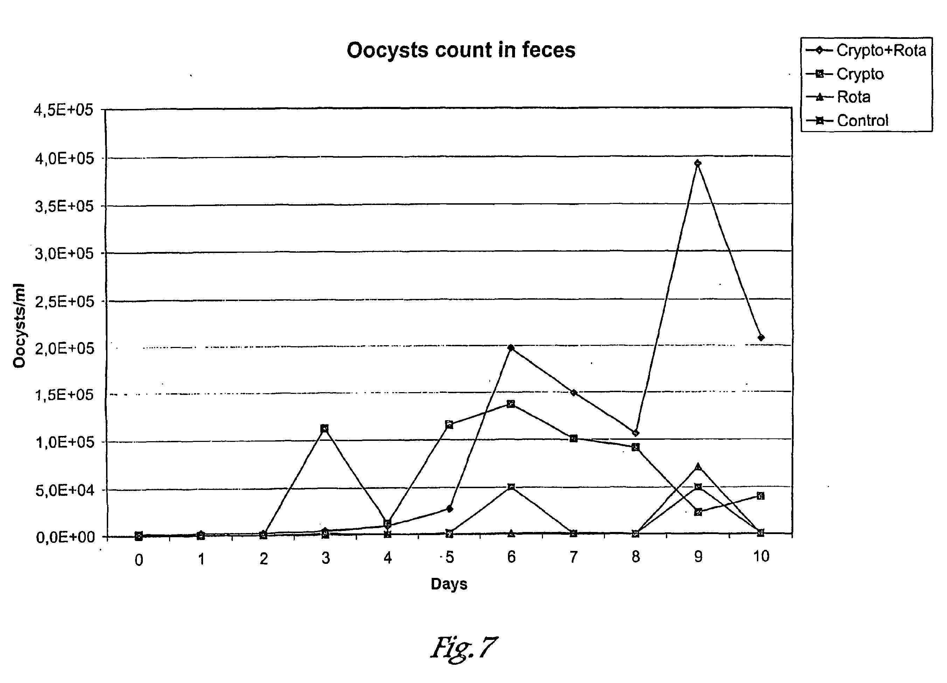

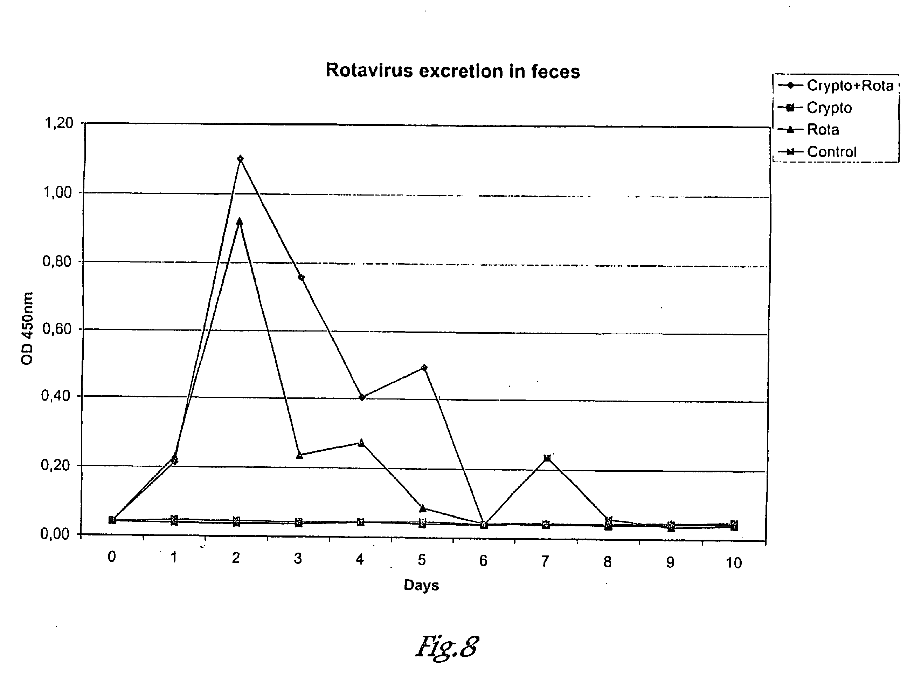

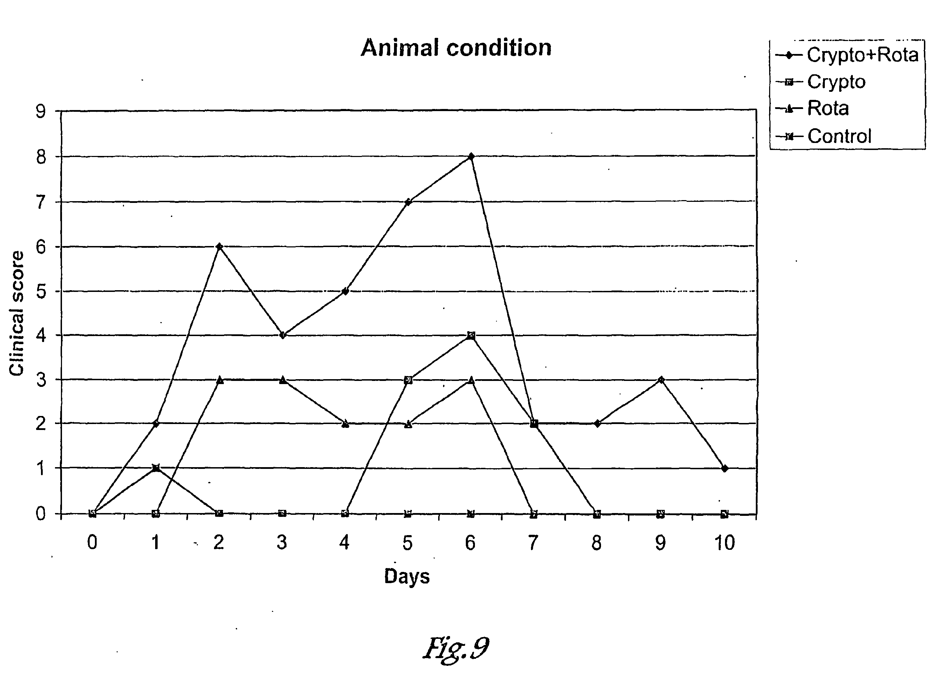

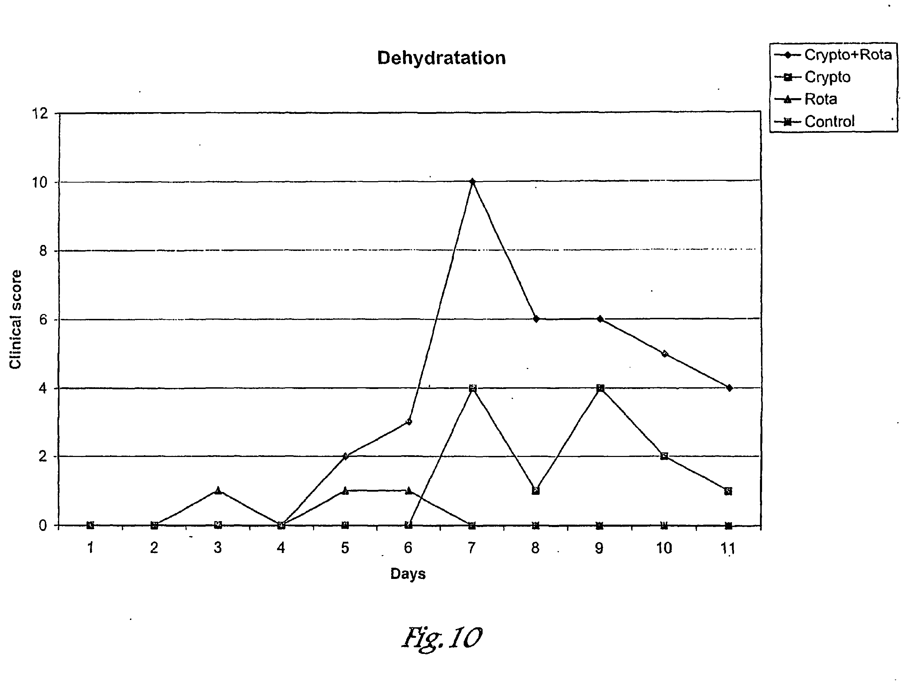

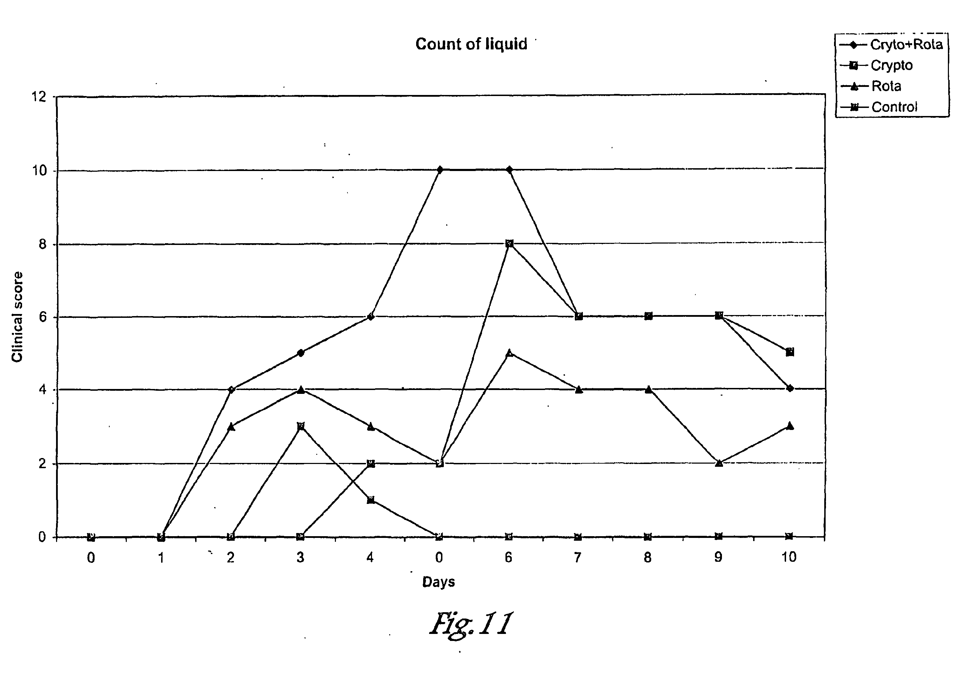

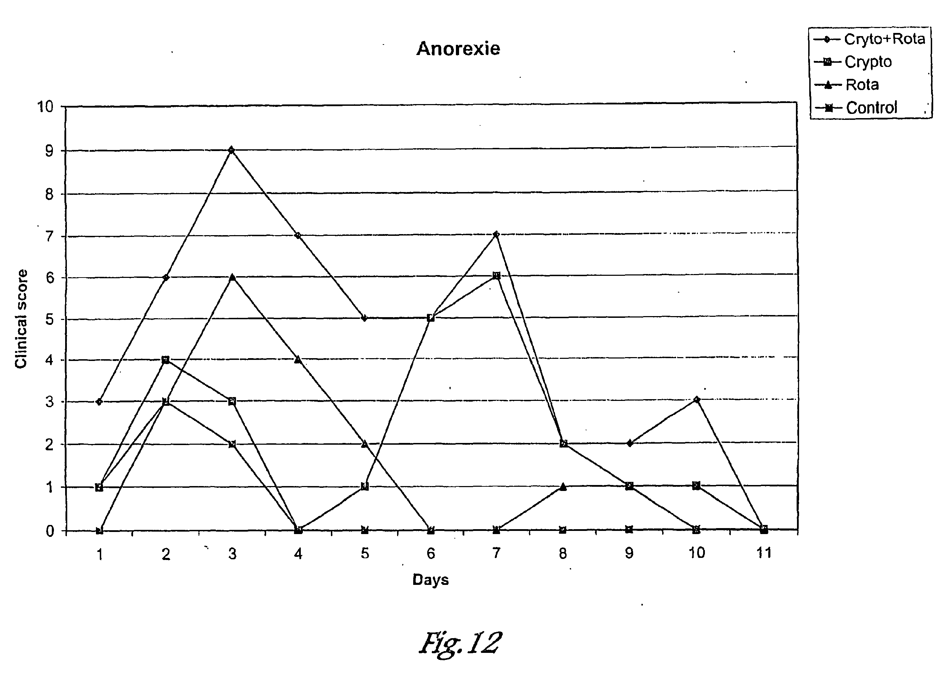

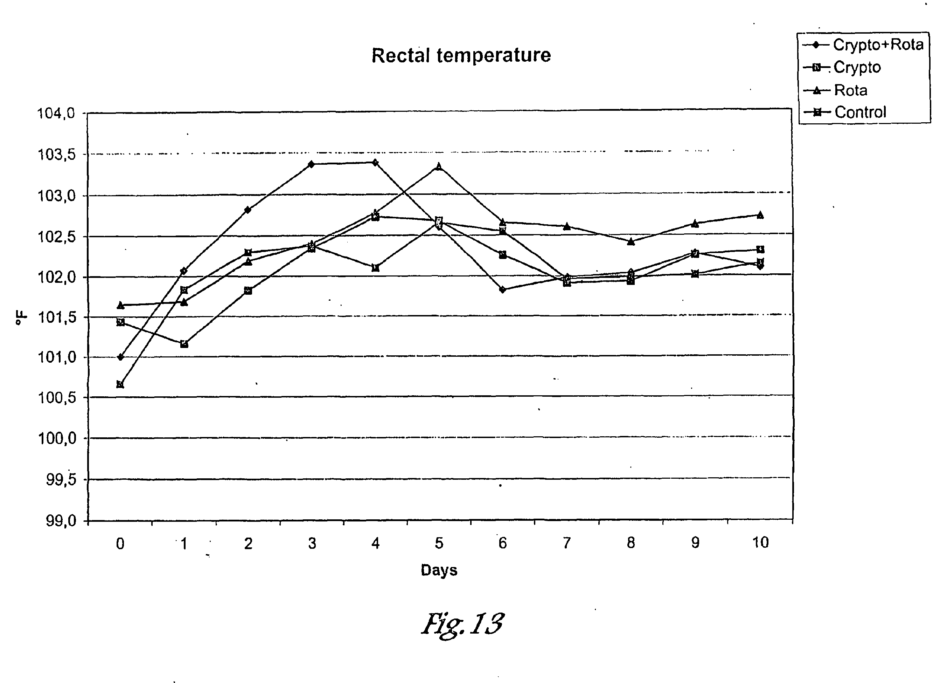

Anorexia is determined based on whether the calf nurses less than 2 liters of milk. During the 1^{st} 48 hours of life, calves may be fed via an esophageal tube. The score is derived for each calf on each day based on the presence of clinical signs (rated 1) or absence (rated 0) for each sickness category.
Rectal temperature is recorded in degrees Farhenheit.
Two calves died in Group C on days 7 and 8, two in Group B on day 7, none in Group D and one in Group A on day 3.
Results are shown on Figures 7 to 13.
A synergistic effect on clinical signs and microorganisms excretion in feces is observed when both microorganisms are administered compare to single administrations.

### REFERENCES

Tzipori S. The relative importance of enteric pathogens affecting neonates of domestic animals. Adv Vet Sci Comp Med, 290:103-206. 1985
Angus K W. Cryptosopridiosis in Ruminants. In: Cryptosopridiosis of man and animals (Edited by Dubey J P, Speer C A & Fayer R), pp 83-103. CRC Press, Boston. 1990.
De la Fuente R; Luz´on M; Ruiz-Santa-Quiteria JA; Garc´ia A; Cid D; Orden JA; Garc´ia S; Sanz R; G'omez-Bautista M. Cryptosporidium and concurrent infections with other major enterophatogens in 1 to 30-day-old diarrheic dairy calves in central Spain. Vet Parasitol, 80(3):179-85. 1999.
Moon HW; McClurkin AW; Isaacson RE; Pohlenz J; Skartvedt SM; Gillette KG; Baetz AL. Pathogenic relationships of rotavirus, Escherichia coli, and other agents in mixed infections in calves. J Am Vet Med Assoc, 173(5 Pt 2):577-83 1978 Sep 1
Viring S; Olsson SO; Aleni´us S; Emanuelsson U; Jacobsson SO; Larsson B; Linde N; Uggla A. Studies of enteric pathogens and gamma-globulin levels of neonatal calves in Sweden. Acta Vet Scand, 34(3):271-9 1993
Perryman LE, Kapil SJ, Jones ML, Hunt EL, "Protection of calves against cryptosporidiosis with immune bovine colostrum induced by a Cryptosporidium parvum recombinant protein" Vaccine 17(17):2142-9 (1999, April 23).
Wakelin, D. "Immune response to intestinal parasites: protection, pathology and prophylaxis" Parassitologia 39(4):269-74 (1997, December).
Iochmann, S. et al., "Comparison of the humoral and cellular immune responses to two preparations of Cryptosporidium parvum CP15/60 recombinant protein" Microb. Pathog. 26(6):307-15 (1999 June).
Sagodira, S. et al., "Protection of kids against Cryptosporidium parvum infection after immunization of dams with CP15-DNA" Vaccine 17(19):2346-55 (1999, May 14).
Enriquez FJ et al., "Role of immunoglobulin A monoclonal antibodies against P23 in controlling murine Cryptosporidium parvum infection" Infect Immun 66(9):4469-73 (1998 September).
Harp JA et al., "Strategies for the control of Cryptosporidium parvum infection in calves" J. Dairy Sci 81(1):289-94 (1998 January).
Sreter T. et al., "Attempts to immunize chickens against Cryptosporidium baileyi with C. parvum oocysts and Paracox vaccine" Folia Parasitol (Praha) 44(1):77-80 (1997).
Harp JA et al., "Field testing of prophylactic measures against Cryptosporidium parvum infection in calves in a California dairy herd" Am J Vet Res 57(11):1586-8)1996 November).
Jenkins M. et al., "Serum and colostrum antibody responses induced by jet-injection of sheep with DNA encoding a Cryptosporidium parvum antigen" Vaccine 13(17):1658-64 (1995 December).
Tatalick LM et al., "Attempts to protect severe combined immunodeficient (scid) mice with antibody enriched for reactivity to Cryptosporidium parvum surface antigen-1." Vet Parasitol 58(4):281-90 (1995 July).
Harp JA et al., "Protection of calves with a vaccine against Cryptosporidium parvum" J Parasitol 81(1):54-7 (1995 February).
Dellert SF et al., "Diarrheal disease. Established pathogens, new pathogens, and progress in vaccine development" Gastroenterol Clin North Am 23(4):637-54 (1994 December).
Bellinzoni RC et al., "Efficacy of an inactivated oil-adjuvanted rotavirus vaccine in the control of calf diarrhoea in beef herds in Argentina" Vaccine 7(3):263-8 (1989 June).
Harp JA et al., "Field testing of prophylactic measures against Crytosporidium parvum infection in calves in a California dairy herd" Am J Vet Res 57(11):1586-8 (1996 November).
Harp JA et al., "Resistance of calves to Cryptosporidium parvum: effects of age and previous exposure" 58(7):2237-40 (1990 July).
Fayer R. et al., "Efficacy of hyperimmune bovine colostrum for prophylaxis of cryptosporidiosis in neonatal calves" J Parasitol 75(3)393-7 (1989 June).
Mosier DA et al., "Bovine humoral immune response to Cryptosporidium parvum" J. Clinical Microbiol 30(12):3277-9 (1992 December).
Sagodira S. et al., "Protection of kids against Cryptosporidium parvum infection after immunization of dams with CP15-DNA" 17(19):2346-55 (1999 May 14)
Finch GR et al., "Dose response of Cryptosporidium parvum in outbred neonatal CD-1 mice" Appl Environ Microbiol 59(11):3661-5 (1993 November).
Jenkins MC et al., "Hyperimmune bovine colostrum specific for recombinant Cryptosporidium parvum antigen confers partial protection against cryptosporidiosis in immunosuppressed adult mice" Vaccine 17(19):2453-60 (1999 May).
Avila FA et al., "A comparative study of the efficiency of a pro-biotic and the anti-K99 and anti-A14 vaccines in the control of diarrhea in calves in Brazil" Rev Elev Med Vet pays Trop 48(3):239-43 (1995).
Castrucci G., "Field trial evaluation of an inactivated rotavirus vaccine against neonatoal diarrhea of calves" Eur J Epidemiol 3(1):5-9 (1987 March).
Perryman LE ct al.. "Immunotherapy of cryptosporidiosis in immunodeficient animal models" J. Protozool 38(6):98S-100S (1991 Nov-Dec).
Yano T. et al.. "Determination of the efficiency of K99-F41 fimbrial antigen vaccine in newborn calves" Braz J. Med. Biol. Res. 28(6):651-4 (1995 June).
Kadel WL et al., "Field-trial evaluation of a Pasteurella vaccine in preconditioned and nonpreconditioned lightweight calves" Am. J. Vet. Res. 46(9): 1944-8 (1985 September).
Kharalambivev KhE et al.. "Attenuated vaccine against rota- and coronavirus enteritis in calves" Vet. Med. Nauki 23(10):26-31 (1986).
Thurber ET ct al., "Field trial evaluation of a reo-coronavirus calf diarrhea vaccine" Can J. Comp Med 41(2):131-6 (1977 April).
Jeff B. Wilson et al., "A Case-control Study of Selected Pathogens Including Verocytotoxigenic Escherichia coli in Calf Diarrhea on an Ontario Veal Farm" Can J. Res 56:184-188 (1992).
J. De Rycke et al., "Prevalence of Various Enteropathogens in the Feces Of Diarrheic And healthy Calves" Ann. Rech. Vet. 17(2): 159-168 (1986).
D.J. Reynolds, et al., "Microbiology of Calf diarrhoea in Southern Britain" Veterinary Record 119:34-39 (1986).
Jorge W. Lopez et al., "Rotavirus and Cryptosporidium Shedding in Dairy Calf Feces and Its Relationship to Colostrum Immune Transfer" J. Dairy Science 71:1288-1294 (1988).
H.W. Moon, DVM, PhD. et al., "Pathogenic Relationships of Rotavirus, Escherichia coli, and Other Agents in Mixed Infections in Calves" JAVMA (September 1, 1978).
S. Virigng et al., "Studies of Enteric Pathogens and -Globulin Levels of Neonatal Calves in Sweden" Acta Vet. Scand. Vol. 34:271-279 (1993).
R. de la Fuenta et al., "Cryptosporidium and concurrent infections with other major enterophatogens in 1 to 30-day-old diarrheic dairy calves in central Spain" Veterinary Parasitology 80:179-185 (1999).
F. Bürki et al., "Reduction of Rotavirus-, Coronavirus- and E. coli-Associated Calf-Diarrheas in a Large-Size Dairy Herd" J. Vet. Med. B. 33, 241-252 (1986).
R. de la Fuente et al., "Proportional morbidity rates of enteropathogens among diarrheic dairy calves in central Spain" Preventive Veterinary Medicine 36:145-152 (1998).
Saul Tzipori, "The Relative Imporatance of Enteric Pathogens Affecting Neonates of Domestic Animals" Advances Veterinary Science and Comparative Medicine, Vol. 29.
Kenneth W. Angus, "Cryptosporidiosis In Ruminants" Cryptosporidiosis of Man and Animals, Vol. 5.
Robert E. Holland, "Some Infectious Causes of Diarrhea in Young Farm Animals" Clincal Microbiology Reviews, p. 345-375 (1990 October).
Perryman et al., "Neutralization-Sensitive Epitopes of Cryptosporidium parvum" PCT WO 98/07320.
Jenkins et al., "DNA Sequence Encoding Surface Protein Of Cryptosporidium Parvum" U.S. Patent Number 5,591,434.

## Claims

1. A combined enteric immunological or vaccine composition comprising:
a first antigen or epitope of interest from *Cryptosporidium parvum* and/or a vector that expresses the first antigen or epitope of interest, wherein said first antigen or epitope of interest is one or more sub-unit *Cryptosporidium parvum* antigens selected from the group consisting of P21, Cp23, Cp 15/60, CP41 and mixtures thereof,
and a second antigen or epitope of interest from another enteric pathogen and/or the first vector that expresses the first antigen or epitope of interest also expresses the second antigen or epitope of interest and/or a second vector that expresses the second antigen or epitope of interest,
and a pharmaceutically acceptable vehicle;
wherein the antigen from the enteric pathogen is selected from the group consisting of the antigens from *E. coli*, rotavirus, coronavirus, and mixtures thereof.

2. The composition according to claim 1 wherein the enteric pathogen comprises *E. coli*.

3. The composition according to claim 2, wherein the antigen from *E. coli* comprises an antigen selected from the group consisting of inactivated *E. coli* bearing K99 antigen, inactivated *E. coli* bearing F41 antigen, inactivated *E. coli* bearing Y antigen, inactivated *E. coli* bearing 31A antigen, K99 antigen, F41 antigen, Y antigen, 31A antigen, and mixtures thereof.

4. The composition according to claim 3 wherein the *E. coli* antigen comprises a K99 antigen selected from the group consisting of inactivated *E. coli* bearing the K99 antigen, K99 antigen, and mixtures thereof; and/or a F41 antigen selected from the group consisting of inactivated *E. coli* bearing the F41 antigen, F41 antigen, and mixtures thereof.

5. The composition according to claim 3 wherein the *E. coli* antigen comprises a K99 antigen selected from the group consisting of inactivated *E. coli* bearing the K99 antigen, K99 antigen, and mixtures thereof; and a F41 antigen selected from the group consisting of inactivated *E. coli* bearing the F41 antigen, F41 antigen, and mixtures thereof; and a Y antigen selected from the group consisting of inactivated *E. coli* bearing the Y antigen, Y antigen, and mixtures thereof.

6. The composition according to claim 1 wherein the enteric pathogen comprises bovine coronavirus.

7. The composition according to claim 1 wherein the enteric pathogen comprises bovine rotavirus.

8. The composition according to claim 1 wherein the enteric pathogen comprises *E. coli,* bovine rotavirus, and bovine coronavirus.

9. The composition according to claim 8, wherein the antigen of the enteric pathogen comprises one or more *E. coli* antigens selected from the group consisting of inactivated *E. coli* bearing K99 antigen, inactivated *E. coli* bearing F41 antigen, inactivated *E. coli* bearing *Y* antigen, inactivated *E. coli* bearing 31 A antigen, K99 antigen, F41 antigen, Y antigen, 31A antigen, and mixtures thereof; inactivated bovine coronavirus; or one or more *E. coli* antigens selected from the group consisting of inactivated *E. coli* bearing K99 antigen, inactivated *E. coli* bearing F41 antigen, inactivated *E. coli* bearing *Y* antigen, inactivated *E. coli* bearing 31A antigen, K99 antigen, F41 antigen, Y antigen, 31A antigen and mixtures thereof; inactivated bovine coronavirus; and inactivated bovine rotavirus.

10. The composition according to claim 9 wherein said one or more *E. coli* antigens comprises a K99 antigen selected from the group consisting of inactivated *E. coli* bearing the K99 antigen, K99 antigen, and mixtures thereof; and/or a F41 antigen selected from the group consisting of inactivated *E. coli* bearing the F41 antigen, F41 antigen, and mixtures thereof.

11. The composition according to claim 9 wherein the *E. coli* antigen comprises a K99 antigen selected from the group consisting of inactivated *E. coli* bearing the K99 antigen, K99 antigen, and mixtures thereof; and a F41 antigen selected from the group consisting of inactivated *E. coli* bearing the F41 antigen, F41 antigen, and mixtures thereof; and a Y antigen selected from the group consisting of inactivated *E. coli* bearing the Y antigen, Y antigen, and mixtures thereof.

12. The composition according to claim 9 wherein said composition comprises *E. coli* antigens K99 and F41.

13. The composition according to any one of claims 1 to 12 wherein said first antigen or epitope of interest is Cp23 and Cp15/60.

14. The composition according to any one of claims 1 to 12 wherein said first antigen or epitope of interest is P21 and Cp15/60.

15. The composition according to any one of claims 1 to 14, which comprises an adjuvant.

16. The composition according to claim 15, wherein the adjuvant comprises saponin or aluminium hydroxide.

17. The composition according to claim 15, wherein the adjuvant is in the form of an oil-in-water emulsion.

18. The composition according to any one of claims 1 to 17 wherein said composition is used as a bovine, canine, feline or equine combined enteric immunological or vaccine composition.

19. Use of a composition according to any one of claims 1 to 17 in the preparation of a medicament for the prevention and/or treatment and/or control of *Cryptosporidium parvum* and/or enteric infections in animals.

20. Use according to claim 19 wherein said animal is a bovine animal.

21. A method for preparing a composition according to any one of claims 1 to 17 comprising admixing the antigens or epitopes or vectors and a carrier.

22. A kit for preparing a composition according to any one of claims 1 to 17 comprising the antigens, epitopes or vectors each in separate container or containers, optionally packaged together; and further optionally with instructions for admixture and/or administration.

## Patentansprüche

1. Kombinierte enterische immunologische Zusammensetzung oder Impfstoffzusammensetzung umfassend:
ein erstes Antigen oder Epitop von Interesse aus *Cryptosporidium parvum* und/oder einen Vektor, welcher das erste Antigen oder Epitop von Interesse exprimiert, wobei das erste Antigen oder Epitop von Interesse eine oder mehrere Untereinheiten von *Cryptosporidium parvum*-Antigenen ausgewählt aus der Gruppe bestehend aus P21, Cp23, Cp15/60, CP41 und Gemischen davon ist/sind,
und ein zweites Antigen oder Epitop von Interesse von einem anderen enterischen Pathogen und/oder den ersten Vektor, welcher das erste Antigen oder Epitop von Interesse sowie das zweite Antigen oder Epitop von Interesse exprimiert, und/oder einen zweiten Vektor, welcher das zweite Antigen oder Epitop von Interesse exprimiert, und einen pharmazeutisch verträglichen Träger;
wobei das Antigen des enterischen Pathogens ausgewählt ist aus der Gruppe bestehend aus den Antigenen von *E. coli,* Rotavirus, Coronavirus und Gemischen davon.

2. Zusammensetzung nach Anspruch 1, wobei das enterische Pathogen *E. coli* umfasst.

3. Zusammensetzung nach Anspruch 2, wobei das Antigen von *E. coli* ein Antigen umfasst, ausgewählt aus der Gruppe bestehend aus inaktiviertem *E. coli,* welches das K99-Antigen trägt, inaktiviertem *E. coli,* welches das F41-Antigen trägt, inaktiviertem *E. coli,* welches das Y-Antigen trägt, inaktiviertem *E. coli*, welches das 31A-Antigen trägt, K99-Antigen, F41-Antigen, Y-Antigen, 31A-Antigen und Gemischen davon.

4. Zusammensetzung nach Anspruch 3, wobei das *E. coli*-Antigen ein K99-Antigen umfasst, ausgewählt aus der Gruppe bestehend aus inaktiviertem *E. coli*, welches das K99-Antigen trägt, K99-Antigen und Gemischen davon; und/oder ein F41-Antigen, ausgewählt aus der Gruppe bestehend aus inaktiviertem *E. coli,* welches das F41-Antigen trägt, F41-Antigen und Gemischen davon.

5. Zusammensetzung nach Anspruch 3, wobei das *E. coli*-Antigen ein K99-Antigen umfasst, ausgewählt aus der Gruppe bestehend aus inaktiviertem *E. coli,* welches das K99-Antigen trägt, K99-Antigen und Gemischen davon; und einem F41-Antigen, ausgewählt aus der Gruppe bestehend aus inaktiviertem *E. coli,* welches das F41-Antigen trägt, F41-Antigen und Gemischen davon; und einem Y-Antigen, ausgewählt aus der Gruppe bestehend aus inaktiviertem *E. coli,* welches das Y-Antigen trägt, Y-Antigen und Gemischen davon.

6. Zusammensetzung nach Anspruch 1, wobei das enterische Pathogen Rinder-Coronavirus umfasst.

7. Zusammensetzung nach Anspruch 1, wobei das enterische Pathogen Rinder-Rotavirus umfasst.

8. Zusammensetzung nach Anspruch 1, wobei das enterische Pathogen *E. coli,* Rinder-Rotavirus und Rinder-Coronavirus umfasst.

9. Zusammensetzung nach Anspruch 8, wobei das Antigen des enterischen Pathogens ein oder mehrere *E. coli*-Antigene umfasst, ausgewählt aus der Gruppe bestehend aus inaktiviertem *E. coli,* welches das K99-Antigen trägt, inaktiviertem *E. coli,* welches das F41-Antigen trägt, inaktiviertem *E. coli,* welches das Y-Antigen trägt, inaktiviertem *E. coli*, welches das 31A-Antigen trägt, K99-Antigen, F41-Antigen, Y-Antigen, 31A-Antigen und Gemischen davon; inaktiviertes Rinder-Coronavirus; ein oder mehrere *E. coli*-Antigene, ausgewählt aus der Gruppe bestehend aus inaktiviertem *E. coli,* welches das K99-Antigen trägt, inaktiviertem *E. coli,* welches das F41-Antigen trägt, inaktiviertem *E. coli,* welches das Y-Antigen trägt, inaktiviertem *E. coli*, welches das 31A-Antigen trägt, K99-Antigen, F41-Antigen, Y-Antigen, 31A-Antigen und Gemischen davon; inaktiviertes Rinder-Coronavirus; und inaktiviertes Rinder-Rotavirus.

10. Zusammensetzung nach Anspruch 9, wobei das eine oder mehrere *E. coli*-Antigen(e) ein K99-Antigen umfasst(en), ausgewählt aus der Gruppe bestehend aus inaktiviertem *E. coli,* welches das K99-Antigen trägt, K99-Antigen und Gemischen davon; und/oder ein F41-Antigen, ausgewählt aus der Gruppe bestehend aus inaktiviertem *E. coli,* welches das F41-Antigen trägt, F41-Antigen und Gemischen davon.

11. Zusammensetzung nach Anspruch 9, wobei das *E. coli*-Antigen ein K99-Antigen umfasst, ausgewählt aus der Gruppe bestehend aus inaktiviertem *E. coli*, welches das K99-Antigen trägt, K99-Antigen und Gemischen davon; und ein F41-Antigen, ausgewählt aus der Gruppe bestehend aus inaktiviertem *E. coli,* welches das F41-Antigen trägt, F41-Antigen und Gemischen davon; und ein Y-Antigen, ausgewählt aus der Gruppe bestehend aus inaktiviertem *E. coli*, welches das Y-Antigen trägt, Y-Antigen und Gemischen davon.

12. Zusammensetzung nach Anspruch 9, wobei die Zusammensetzung die *E. coli*-Antigene K99 und F41 umfasst.

13. Zusammensetzung nach einem der Ansprüche 1 bis 12, wobei das erste Antigen oder Epitop von Interesse Cp23 und Cp15/60 ist.

14. Zusammensetzung nach einem der Ansprüche 1 bis 12, wobei das erste Antigen oder Epitop von Interesse P21 und Cp15/60 ist.

15. Zusammensetzung nach einem der Ansprüche 1 bis 14, welche ein Adjuvans umfasst.

16. Zusammensetzung nach Anspruch 15, wobei das Adjuvans Saponin oder Aluminiumhydroxid umfasst.

17. Zusammensetzung nach Anspruch 15, wobei das Adjuvans in Form einer Öl-in-Wasser-Emulsion vorliegt.

18. Zusammensetzung nach einem der Ansprüche 1 bis 17, wobei die Zusammensetzung als kombinierte enterische immunologische Zusammensetzung oder Impfstoffzusammensetzung für Rinder, Hunde, Katzen, oder Pferde verwendet wird.

19. Verwendung einer Zusammensetzung nach einem der Ansprüche 1 bis 17 für die Herstellung eines Medikaments zur Vorbeugung und/oder Behandlung und/oder Kontrolle von *Cryptosporidium parvum-Infektionen* und/oder enterischen Infektionen in Tieren.

20. Verwendung nach Anspruch 19, wobei das Tier ein rinderartiges Tier ist.

21. Verfahren zur Herstellung einer Zusammensetzung nach einem der Ansprüche 1 bis 17, umfassend das Zusammenmischen von Antigenen oder Epitopen oder Vektoren und einem Träger.

22. Kit für die Herstellung einer Zusammensetzung nach einem der Ansprüche 1 bis 17, umfassend die Antigene, Epitope oder Vektoren, welche in einem separaten Behälter oder Behältern vorliegen, und welche gegebenenfalls zusammengepackt sind; und weiterhin gegebenenfalls mit Anweisungen für das Zusammenmischen und/oder die Verabreichung.

## Revendications

1. Composition immunologique ou vaccinale entérale combinée comprenant :
un premier antigène ou épitope d'intérêt issu de *Cryptosporidium parvum* et/ou un vecteur qui exprime le premier antigène ou épitope d'intérêt, dans laquelle ledit premier antigène ou épitope d'intérêt est une ou plusieurs sous-unité(s) de *Cryptosporidium parvum* choisie(s) dans le groupe constitué de P21, Cp23, Cp15/60, Cp41 et leurs mélanges,
et un second antigène ou épitope d'intérêt issu d'un autre agent pathogène entérique et/ou le premier vecteur qui exprime le premier antigène ou épitope d'intérêt et qui exprime aussi le second antigène ou épitope d'intérêt et/ou un second vecteur qui exprime le second antigène ou épitope d'intérêt,
et un véhicule pharmaceutiquement acceptable ;
dans laquelle l'antigène issu de l'agent pathogène entérique est choisi dans le groupe constitué des antigènes de *E. coli,* de rotavirus, de coronavirus, et leurs mélanges.

2. Composition selon la revendication 1 dans laquelle l'agent pathogène entérique comprend *E. coli.*

3. Composition selon la revendication 2, dans laquelle l'antigène de *E. coli* comprend un antigène choisi dans le groupe constitué de *E. coli* inactivé portant l'antigène K99, *E. coli* inactivé portant l'antigène F41, *E. coli* inactivé portant l'antigène Y, *E. coli* inactivé portant l'antigène 31A, l'antigène K99, l'antigène F41, l'antigène Y, l'antigène 31A, et leurs mélanges.

4. Composition selon la revendication 3, dans laquelle l'antigène de *E. coli* comprend un antigène K99 choisi dans le groupe constitué de *E. coli* inactivé portant l'antigène K99, l'antigène K99, et leurs mélanges ; et/ou un antigène F41 choisi dans le groupe constitué de *E. coli* inactivé portant l'antigène F41, l'antigène F41, et leurs mélanges.

5. Composition selon la revendication 3 dans laquelle l'antigène de *E. coli* comprend un antigène K99 choisi dans le groupe constitué de *E. coli* inactivé portant l'antigène K99, l'antigène K99, et leurs mélanges ; et un antigène F41 choisi dans le groupe constitué de *E. coli* inactivé portant l'antigène F41, l'antigène F41, et leurs mélanges ; et un antigène Y choisi dans le groupe constitué de *E. coli* inactivé portant l'antigène Y, l'antigène Y, et leurs mélanges.

6. Composition selon la revendication 1, dans laquelle l'agent pathogène entérique comprend le coronavirus bovin.

7. Composition selon la revendication 1, dans laquelle l'agent pathogène entérique comprend le rotavirus bovin.

8. Composition selon la revendication 1, dans laquelle l'agent pathogène entérique comprend *E. coli*, le rotavirus bovin et le coronavirus bovin.

9. Composition selon la revendication 8, dans laquelle l'antigène de l'agent pathogène entérique comprend un ou plusieurs antigènes de *E. coli* choisis dans le groupe constitué de *E. coli* inactivé portant l'antigène K99, *E. coli* inactivé portant l'antigène F41, *E. coli* inactivé portant l'antigène Y, *E. coli* inactivé portant l'antigène 31A, l'antigène K99, l'antigène F41, l'antigène Y, l'antigène 31A, et leurs mélanges, le coronavirus bovin inactivé ; ou un ou plusieurs antigènes de *E. coli* choisis dans le groupe constitué de *E. coli* inactivé portant l'antigène K99, *E. coli* inactivé portant l'antigène F41, *E. coli* inactivé portant l'antigène *Y, E. coli* inactivé portant l'antigène 31A, l'antigène K99, l'antigène F41, l'antigène Y, l'antigène 31A et leurs mélanges, le coronavirus bovin inactivé et le rotavirus bovin inactivé.

10. Composition selon la revendication 9, dans laquelle ledit antigène ou lesdits antigènes de *E. coli* comprennent un antigène K99 choisi dans le groupe constitué de *E. coli* inactivé portant l'antigène K99, l'antigène K99, et leurs mélanges ; et/ou un antigène F41 choisi dans le groupe constitué de *E. coli* inactivé portant l'antigène F41, l'antigène F41, et leurs mélanges.

11. Composition selon la revendication 9, dans laquelle l'antigène de *E. coli* comprend un antigène K99 choisi dans le groupe constitué de *E. coli* inactivé portant l'antigène K99, l'antigène K99 et leurs mélanges ; et un antigène F41 choisi dans le groupe constitué de *E. coli* inactivé portant l'antigène F41, l'antigène F41 et leurs mélanges ; et un antigène Y choisi dans le groupe constitué de *E. coli* inactivé portant l'antigène Y, l'antigène Y et leurs mélanges.

12. Composition selon la revendication 9, dans laquelle ladite composition comprend les antigènes de *E. coli* K99 et F41.

13. Composition selon l'une quelconque des revendications 1 à 12, dans laquelle ledit premier antigène ou épitope d'intérêt est Cp23 et Cp15/60.

14. Composition selon l'une quelconque des revendications 1 à 12, dans laquelle ledit premier antigène ou épitope d'intérêt est P21 et Cp15/60.

15. Composition selon l'une quelconque des revendications 1 à 14, qui comprend un adjuvant.

16. Composition selon la revendication 15, dans laquelle l'adjuvant comprend de la saponine ou de l'hydroxyde d'aluminium.

17. Composition selon la revendication 15, dans laquelle l'adjuvant est sous la forme d'une émulsion huile dans eau.

18. Composition selon l'une quelconque des revendications 1 à 17 dans laquelle ladite composition est utilisée comme composition immunologique ou vaccinale entérique combinée bovine, canine, féline ou équine.

19. Utilisation d'une composition selon l'une quelconque des revendications 1 à 17 dans la préparation d'un médicament pour la prévention et/ou le traitement et/ou le contrôle des infections par Cryptosporidium parvum et/ou des infections entériques chez les animaux.

20. Utilisation selon la revendication 19 dans laquelle l'animal est un bovin.

21. Procédé pour préparer une composition selon l'une quelconque des revendications 1 à 17 comprenant le mélange des antigènes ou épitopes ou vecteurs et d'un véhicule.

22. Kit pour préparer une composition selon l'une quelconque des revendications 1 à 17 comprenant les antigènes, les épitopes et les vecteurs, chacun dans un ou des récipients distincts, facultativement emballés ensemble, et, en outre, facultativement des instructions pour le mélangeage et/ou l'administration.


### SEQUENCE LISTING

<110> MERIAL
<120> Compositions and vaccines containing antigen(s) of cryptosporidium pa rvum and of another pathogen
<130> BET 00/1191
<140> PCT/EP00/13387
   <141> 2000-12-20
<150> US 171399
   <151> 1999-12-21
<160> 10
<170> Patentln version 3.0
<210> 1
   <211> 35
   <212> DNA
   <213> Artificial
<220>
   <223> oligonucleotide
<400> 1
   ttttttccat ggggctcgag ttttcgcttg tgttg 35
<210> 2
   <211> 33
   <212> DNA
   <213> Artificial
<220>
   <223> oligonucleotide
<400> 2
   ttttttgaat tcttaggcat cagctggctt gtc 33
<210> 3
   <211> 35
   <212> DNA
   <213> Artificial
<220>
   <223> oligonucleotide
<400> 3
   ttttttctcg agatgggtaa cttgaaatcc tgttg 35
<210> 4
   <211> 42
   <212> DNA
   <213> Artificial
<220>
   <223> oligonucleotide
<400> 4
   ttttttgaat tcttagttaa agtttggttt gaatttgttt gc 42
<210> 5
   <211> 35
   <212> DNA
   <213> Artificial
<220>
   <223> oligonucleotide
<400> 5
   ttttttccat ggggtcccct atactaggtt attgg 35
<210> 6
   <211> 45
   <212> DNA
   <213> Artificial
<220>
   <223> oligonucleotide
<400> 6
   ttttttctcg agcctgcagc ccggggatcc aacagatgca cgacg 45
<210> 7
   <211> 33
   <212> DNA
   <213> Artificial
<220>
   <223> oligonucleotide
<400> 7
   ttttttctcg agttttcgct tgtgttgtac agc 33
<210> 8
   <211> 65
   <212> DNA
   <213> Artificial
<220>
   <223> oligonucleotide
<400> 8
   ttttttccat ggggggttct catcatcatc atcatcatgg tctcgagttt tcgcttgtgt 60
   tgtac 65
<210> 9
   <211> 64
   <212> DNA
   <213> Artificial
<220>
   <223> oligonucleotide
<400> 9
   ttttttccat ggggggttct catcatcatc atcatcatgg tatgggtaac ttgaaatcct 60
   gttg 64
<210> 10
   <211> 31
   <212> DNA
   <213> Artificial
<220>
   <223> oligonucleotide
<400> 10
   ttttttccat gggtaacttg aaatcctgtt g 31